# EUROPEAN PATENT SPECIFICATION

(11) **EP 4 637 856 B1**
(45) Date of publication and mention of the grant of the patent: **25.02.2026**
(21) Application number: 24820747.4
(22) Date of filing: 06.12.2024
(51) Int. Cl.: A61L 31/02, A61L 31/10, A61L 31/14, A61L 31/16, A61L 31/18

(54) **VASCULAR SCAFFOLD HAVING A RARE-EARTH METAL RADIOPAQUE MARKER**
GEFÄSSGERÜST MIT RÖNTGENDICHTEM SELTENERDMETALLMARKER
ÉCHAFAUDAGE VASCULAIRE AYANT UN MARQUEUR RADIO-OPAQUE DE MÉTAL DE TERRES RARES

(30) Priority: 08.12.2023 EP 23215271
(43) Date of publication of application: 29.10.2025
(73) Proprietor: MeKo Manufacturing e.K., 31157 Sarstedt (DE)
(72) Inventor: DOHSE, Jakob, 30559 Hannover (DE); MENZE, Roman, 30966 Hemmingen (DE)
(74) Representative: Arth, Hans-Lothar
(86) International application number: PCT/EP2024/085093
(87) International publication number: WO 2025/120167

(56) References cited:
- US-A1- 2019 269 532
- US-A1- 2020 139 017
- US-B2- 8 871 829

## Description

The present invention relates to vascular scaffolds made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, and wherein the vascular scaffold is completely covered with an inorganic coating comprising magnesium fluoride. The vascular scaffolds according the invention can additionally be coated with an organic coating and/or with at least one antiinflammatory, antiproliferative, antiangiogenic, antirestenotic, and/or antithrombogenic active agent.

Nowadays, the implantation of vessel grafts such as stents is a common surgical intervention for the treatment of stenoses. Stents are usually made of metal alloys such as stainless steel or nitinol. Such metal stents are known in large numbers and have proven to work in practice. Due to their metallic structure and load capacity such metal stents should ensure that the vessels remain open after implantation and that the blood flow through the vessels will be ensured permanently.

However, recent investigations have shown that vascular stenoses do not have to be dilated permanently by means of an endoprosthesis, particularly in form of a stent. It is absolutely sufficient to support the blood vessel temporarily as the traumatized tissue of the vessel heals and the smooth muscle cells of the vessel regenerate and resume the task of keeping the blood vessel open, and thus the vessel graft does not need to remain longer than is necessary in the vessel lumen.

Vessel grafts are currently divided into two basic types, permanent as well as degradable or resorbable grafts. Permanent grafts are stents which are designed such that they can remain in the vessel for an indefinite period of time. Resorbable grafts are scaffolds, which are degraded in the vessel or the vessel wall over a predetermined period of time.

Currently, one tries to solve the problem of restenosis after graft implantation by attempting to locally inhibit the growth of the smooth muscle cells. This is for example attempted with stents, which release pharmaceutically active agents, preferably having an antiproliferative effect. These active agents are mostly released from an active agent-containing coating, which may be applied both to permanent and to resorbable stents.

The supporting effect by the metal structure is often only required for short periods of time as the body tissue can recover after the implantation of the stent and the supporting function is no longer necessary. Preferably, degradable and resorbable scaffolds are only degraded when the traumatized tissue of the vessel has healed and the vessel re-stabilized, so that the scaffold does no longer have to remain in the vessel lumen. Especially in the case of scaffolds coming in contact with blood, these cause as being a material foreign to the body a reaction of the surrounding tissue which can result in the formation of restenosis through proliferation. Efforts in the development of scaffolds to an improved biocompatibility of the scaffold material, greater flexibility with decreasing material fatigue and reduction of the foreign surface should minimize the risk of stent-induced restenosis rate further and further. Here, resorbable scaffolds have the advantage that the material foreign to the body does not permanently remain in the vessel and the risk of restenosis is therefore temporally limited. A use of resorbable scaffolds is advantageous for children too, since vascular growth is not adversely affected, or the scaffold does not need to be removed again after a while during which the child has grown.

For said reason, scaffolds consisting of bioresorbable materials, such as for example of polymers such as polyhydroxybutyrate or of metals such as magnesium or iron are increasingly developed in recent times and used in clinical trials.

The large restoring forces of vessels in the first days after dilation are a major cause of restenosis. Therefore, resorbable vessel grafts must consist of a material which can be degraded well by the body, but also has a sufficiently high retention force to prevent restenosis of the vessel.

Once inserted, a scaffold must maintain its size and shape despite the different forces acting on it such as the pulsating load by the beating heart. In addition, the scaffold must have sufficient flexibility to be crimped onto a balloon, and later to be expanded in the vessel.

Resorbable polymers which are used for the production of grafts have lower mechanical strength than those non-resorbable metal alloys used hitherto. A compensation of this disadvantage can be achieved by greater strut widths of the graft. However, this increases the mechanical irritation of the vessel wall during graft implantation, and thus also the risk of restenosis. Resorbable scaffolds from iron or an iron-based alloy have the disadvantage that the residence time in the vessel up to the full degradation is longer than necessary and desired. For resorbable scaffolds the desired period of resorption is between 3 and 12 months, wherein the mechanical load capacity needs to be ensured before. Magnesium is an abundant mineral present in the body and therefore suitable as a basis for a resorbable scaffold. Further, alloying elements were selected from the group of the rare earths, as these do not naturally occur in the body. This allows a detection of the degradation products in the tissue and in the organs.

Magnesium and magnesium alloys have excellent mechanical and physical properties for a wide range of applications. Their low weight along with high strength qualifies magnesium and magnesium alloys as appropriate materials also for endoprostheses. Magnesium and magnesium alloys are very reactive and therefore susceptible to corrosion. Nevertheless these properties are desirable for absorbable implants. However, the following problems exist in the prior art: Although in principle the objective of resorption of the implanted scaffold is achieved, the problem of a temporally non-defined degradation of the scaffold exists. Depending on the choice of material, the degradation of the material is subjected to strong fluctuations, cannot be controlled, and is generally too fast to ensure a safe ingrowth of the scaffold into the vessel walls. When resorption occurs too fast the resorbable scaffold cannot grow into the vessel wall and take over the supporting function until the regeneration of the vessel segment. It can rather detach, or fragments of the scaffold may be detached and be swept away in the blood flow and cause life-threatening problems for the patient.

A bioresorbable metal endoprosthesis of magnesium and yttrium is disclosed in the European patent application EP 1 419 793 A1. A magnesium alloy with yttrium, neodymium and further optional components suitable for the production of implants is described in the European patent application EP 2 169 090 A1. In clinical trials, these implants demonstrated safety and efficacy profile comparable to drug eluting stents, even in complex lesions. However, the bioresorbable implants have the disadvantage that they dissolve too fast and also uncontrolled. Since the dissolution process usually starts before the implant is grown into the vessel wall, fragments may be detached, transported through the bloodstream and cause a heart attack.

The European patent applications EP 2 213 314 A1 and EP 1 842 507 A1 also disclose stents of a magnesium alloy containing gadolinium. To obtain the desired mechanical properties such as strength, tension force and ductility, gadolinium is required in quantities greater than 5% by weight. At quantities greater than 5% by weight gadolinium, however, the problem arises that the processability of the alloy into a tube suitable for laser processing, and the homogeneity of the alloy is no longer guaranteed. The poorer processability leads to thicker stent struts which are a problem since the bloodstream was obstructed, which has led to thrombi.

An influence on the kinetics of resorption as well as degradation can also be achieved by matching of an optimized material for the basic scaffold and a coating, which positively affects the dissolution kinetics, i.e. slowing down. Such a coating has also to be sufficiently resistant for example to withstand the stresses that a stent is exposed to during implantation without damage.

Magnesium fluoride coatings for grafts made of bioresorbable magnesium alloys are well known in the art. Lin et al. describe a protective effect of the magnesium fluoride film on the JBDM alloy Mg2.5Nd0.2Zn0.4Zr (ACS Applied Materials & Interfaces 2015, 7, p. 5320-5330). The magnesium fluoride film was produced by treating the alloy with a potassium fluoride solution. In vitro studies showed that the degradation rate could be reduced by 20% compared to the uncoated alloy.

Nan et al. investigated the influence of a magnesium fluoride and a collagen coating on the formation of endothelial cells on bioabsorbable magnesium struts (International Journal of Molecular Sciences 2014, 15, p. 5263-5276). The endothelial cell deposition and proliferation were significantly more pronounced on a magnesium fluoride coating than on a collagen coating.

The international patent application WO 2014/143521 A1 discloses stents of bioabsorbable magnesium alloys for which their corrosion was decelerated by passivation using hydrothermal treatment. The corrosion resistance increases with increasing layer thickness. However, layer thicknesses of over 150 µm are necessary for sufficiently long resistance, which can lead to increased crack appearance in the coating due to the dynamic load of the stent. The stents may also have a polymer coating to shield the stent body from the external environment and thus decelerate corrosion.

The international patent application WO 2016/073851 A2 also disclosed stents of a bioabsorbable magnesium alloy, the corrosion of which has been slowed down by passivation by hydrothermal treatment and which have a moisture barrier layer. This additional layer consists of a metal, an organometallic derivative or a polymeric alkoxide such as polyaluminium ethylene glycol (Alucon).

The publication of patent application US 2010/145436 A1 discloses a biodegradable stent of metal or bioabsorbable or biostable polymer having a degradation retardant magnesium fluoride coating or polymer coating. The polymer coating detaches from the stent during expansion so that the stent comes into contact with the vessel wall.

Biostable metallic stents having a porous surface containing an active agent and a non-polymeric biodegradable coating are described in WO 2010/025078 A2. The biodegradable coating of magnesium fluoride, calcium phosphate, apatite, calcium carbonate or calcium fluoride serves for the delayed controlled release of the active agent.

Most biodegradable alloys for medical implants, especially for grafts, known from the related art have the disadvantage that they are not detectable or are only detectable to an unsatisfactory extent in current X -ray methods. However, X-ray visibility or radiopacity is an important property of a stent during placement and deployment, especially in case of stent dislocation or the need for multiple stent placements. Moreover, it is also important for monitoring of the healing progress or for checking minimally invasive interventions by X-ray. Thus, for example, stents have been placed in the coronary arteries during acute myocardial infarction treatment for some years. According to current methods, a catheter which carries the stent or scaffold in an unexpanded state is positioned in the area of the lesion of the coronary vessel wall. Subsequently, the stent expands either through self-expansive forces or through inflation of a balloon, in order to prevent obstruction of the vessel wall in the expanded state. The procedure of positioning and expanding the stent must be monitored continuously during the procedure through interventional cardiology.

The X-ray visibility of an implant manufactured from a metallic material is a function of the thickness of the material and, in addition, of the (mass) attenuation coefficient of the material. Iron as a component of medical steels has a coefficient, which is typically already insufficient for a contrast-rich monitoring image with the filigree structures of stents. Furthermore, it is known that the (mass) attenuation coefficient becomes greater with increasing atomic number in the periodic system.

Providing implants with a coating, a strip, or a marker which is incorporated or moulded on in another way to improve the X-ray visibility is therefore known.

Known marker methods provide attaching metal strips made of gold or other noble metals in specific areas of the stent, for example. Metal strips of this type may loosen, shift, or even fall off, however.

With metal coating methods, radiopaque marking areas may be applied to the grafts through chemical or physical vacuum deposition (CVD or PVD). Alternatively, methods such as ion beam assisted deposition (IBAD) and microfusion are suitable, using which very homogeneous coatings in the micrometer range are producible on the implant surface.

The application of marker layers and the positioning of marker elements on the implants made of biodegradable alloys are anything but trivial and typically requires individual tailoring of the material properties of the marker element to the further materials used in the implant and also adaptations in the design of the implant. Known attempted achievements of the object may therefore not be transferred without further steps to new materials, in particular the promising biodegradable alloys. Furthermore, the marker element itself is to be at least largely biodegradable or is at least to be converted into physiologically harmless components. These are understood as components, which have dimensions significantly smaller than the dimensions of the marker before implantation, but which are not degraded further and are intercalated in the body in unchanged form.

However, local corrosion may be a severe issue when using radiopaque markers in biodegradable grafts. A non-degradable marker made of a noble metal may cause the formation of a local element with the usually non-noble metals of the main body of implant, through which the implant body corrodes and the marker itself may be released. However, when the marker is released very rapidly from the implant body, this may also occur before the complete endothelialisation; the marker may then float away and result in an embolization. Furthermore, there is the danger of abrasion of the intima during positioning of the implant.

The final corrosion behavior of the implant depends on the surface condition of the implant body and the mode of attachment of the marker onto the implant body. Depending on the manufacturing process (such as electropolishing of the implant body, welding or clipping of the markers to the stent corpus) resistance against localized corrosion can be higher or lower.

U.S. patent application US 2019/269532 A1 discloses a biodegradable metal scaffold comprising a plurality of interconnected struts, which comprise an open space in which a radio-opaque marker is disposed.

U.S. patent US 8,871,829 B2 discloses a radiopaque marker for medical implants, which is specifically designed to degrade rapidly. The marker is made of a biodegradable alloy on basis of magnesium, iron or zinc and a radiopaque noble element. The marker alloy reduces tensions at the phase boundaries between the marker and main body of the implant due to the adaptation of the material properties, while accelerating the decomposition of the implant due to formation of local elements in the heterogeneous alloy between the marker component and the alloy base components. A biodegradable implant made of a biodegradable Mg alloy main body having cavities in which the Mg alloy marker is disposed, is also disclosed.

U.S. patent application US 2020/139017 A1 discloses a stent of biodegradable magnesium alloy having an inorganic coating comprising magnesium fluoride and having an organic coating, wherein the magnesium alloy contains at least 80% by weight magnesium, and wherein the inorganic coating covers the stent and the organic coating covers the inorganic coating.

In order to minimise local corrosion, additional measures were taken in the prior art, which makes the manufacturing of medical implants more complex. For instance, in medical implants comprising a magnesium alloy body, tantalum markers are silicon-covered to avoid interactions with the magnesium alloy, because magnesium has great chemical and galvanic sensitivity to direct proximity to different metals.

Therefore, there is a need to develop suitable biodegradable radiopaque vascular scaffolds with optimized corrosion behaviour and which are readily to manufacture.

The object of the present invention is the provision of an easy to manufacture biodegradable radiopaque vascular scaffold.

More specific, the object of the present invention is the provision of an easy to manufacture biodegradable radiopaque vascular scaffold having a minimized galvanic corrosion between the radiopaque marker and the scaffold body.

Said objective is solved by the technical teaching of the independent claims of the present invention. Further advantageous embodiments of the invention result from the dependent claims, the description, and the examples.

Surprisingly, it has been found that vascular scaffolds made of a biodegradable magnesium alloy which hold a pure rare-earth metal as radiopaque marker in a form-fitting or force-fitting manner and which are covered by an inorganic coating comprising magnesium fluoride are not prone to galvanic corrosion between the radiopaque marker and the biodegradable magnesium alloy.

Therefore, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, and wherein the vascular scaffold is completely covered with an inorganic coating comprising magnesium fluoride..

The at least one structure of the inventive vascular scaffold is shaped in such a way that the radiopaque marker is connected in a **form-fitting or force-fitting manner.** To this extent, the at least one structure is shaped so that the radiopaque marker is incorporated into the structure. Therefore, size and shape (i.e. thickness, width, height, diameter etc.) of the at least one structure necessarily correspond to the size and shape of the radiopaque marker to establish a form-fit or force-fit connection. The term "in a form-fitting or force-fitting manner" or "form-fit or force-fit connection" has the meaning herein that the at least one structure holds the radiopaque marker tight such that the radiopaque marker does not release from the structure during and after a medical intervention, including scaffold expansion and dilatation. Particularly, the radiopaque marker does not release from the scaffold body prematurely or before endothelialisation. Due to the different degradation behaviour of the radiopaque marker and the scaffold body, the radiopaque marker is useful for determining the former position of the vascular scaffold in the tissue, for instance by CT scans, even after complete degradation of the vascular scaffold.

Preferably, the at least one structure is shaped so that the radiopaque marker is completely incorporated into the structure. Preferably, the at least one structure is shaped so that the radiopaque marker is at least partially incorporated into the structure. However, the at least one structure of the inventive vascular scaffold is not shaped so that the radiopaque marker is completely encapsulated in the structure.

Thus, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one structure is shaped so that the at least one radiopaque marker is at least partially incorporated into the structure.

In other words, the at least one structure of the inventive vascular scaffold is designed to form a support that surrounds the radiopaque marker at least partially. Thus, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one support structure surrounds the radiopaque marker at least partially.

Reworded, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one structure is shaped circumferentially around the marker.

The at least one structure may be located at any position of the cylindrical scaffold body of the vascular scaffold, including distal end, proximal end, and center part. The at least one structure may be located on a single strut or at the interconnection between the struts. The at least one structure may form an interconnection between two struts.

The at least one structure may be distributed randomly, equally or in a defined pattern on the cylindrical scaffold body of the vascular scaffold.

The at least one structure can be made of the same biodegradable magnesium alloy as the cylindrical scaffold body or can be made of a different biodegradable material. Preferably, the at least one structure is made of the same biodegradable magnesium alloy as the cylindrical scaffold body, thereby forming a monolithic scaffold together with the cylindrical scaffold body.

The at least one structure may have the same thickness as the struts of the cylindrical scaffold body or may have a larger or a smaller thickness. Preferably, the at least one structure and the struts of the cylindrical scaffold body have the same thickness.

In one embodiment, each and every structure of the at least one structure holds a radiopaque marker. In a further embodiment, only some structures of the at least one structure holds a radiopaque marker. In a further embodiment, only two structures of the at least one structure holds a radiopaque marker. In a further embodiment, only three structures of the at least one structure holds a radiopaque marker. In a further embodiment, only four structures of the at least one structure holds a radiopaque marker.

In a preferred embodiment, a first structure of the at least one structure holding a radiopaque marker is located at the distal end of the scaffold and a second structure of the at least one structure holding a radiopaque marker is located at the proximal end of the scaffold. Thus, the present invention also relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein a first structure of the at least one structure holding a radiopaque marker is located at the distal end of the scaffold and a second structure of the at least one structure holding a radiopaque marker is located at the proximal end of the scaffold.

In a preferred embodiment, a first and a second structure of the at least one structure holding a radiopaque marker are located at the distal end of the scaffold and a third and a fourth structure of the at least one structure holding a radiopaque marker are located at the proximal end of the scaffold. Thus, the present invention also relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein a first and a second structure of the at least one structure holding a radiopaque marker are located at the distal end of the scaffold and a third and a fourth structure of the at least one structure holding a radiopaque marker are located at the proximal end of the scaffold.

The at least one structure of the inventive vascular scaffold is designed in the broadest meaning as an opening which can have any shape that is suitable to hold the radiopaque marker in a form-fitting or force-fitting manner. Suitable shapes of said opening include, but are not restricted to, circular, oval, ellipsoidal, angular, angular with bevelled edges, square, rectangular, triangular, pentagonal, hexagonal, heptagonal, octagonal, 9-sided, 10-sided etc.

Thus, in one embodiment, the vascular scaffold made of a biodegradable magnesium alloy comprises a cylindrical scaffold body formed of interconnected struts, wherein the interconnected struts comprise at least one structure designed as an opening to hold a radiopaque marker and wherein the at least one structure holds a rare-earth metal as radiopaque marker in a form-fitting or force-fitting manner.

Another embodiment is directed to a vascular scaffold made of a biodegradable magnesium alloy comprising a cylindrical scaffold body formed of interconnected struts, wherein the interconnected struts comprise at least one structure designed to hold a radiopaque marker, wherein the at least one structure holds the radiopaque marker in a form-fitting or force-fitting manner, wherein the radiopaque marker is dysprosium, and wherein the vascular scaffold is completely covered with an inorganic coating comprising magnesium fluoride.

In another embodiment, the vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the opening has a shape selected from circular, oval, elliptical, angular, angular with bevelled edges, square, rectangular, triangular, pentagonal, hexagonal, heptagonal, octagonal, 9-sided, 10-sided etc.

It has been found that holes are particularly suitable for holding the radiopaque marker in a form-fitting or force-fitting manner. Thus, in a preferred embodiment, the vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one structure is designed as a hole, a blind hole, a through hole, a depression or an eyelet.

Preferably, the at least one structure is a through hole or an eyelet. More preferably, the at least one structure is an elliptical hole, an elliptical through hole or an elliptical eyelet.

In a preferred embodiment, the vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one structure is designed as an elliptical hole, an elliptical through hole or an elliptical eyelet.

It has to be understood that the shape of the radiopaque marker depends on the shape of the at least one structure. Therefore, from the form-fit or force-fit connection of the radiopaque marker and the at least one structure, the skilled person will readily envision that the radiopaque marker has to be brought in a shape which corresponds to the shape of the at least one structure by e.g., laser cutting. For example, an at least one structure having a circular shape can only hold a radiopaque marker having a circular disc shape in a form-fitting or force-fitting manner.

Since the radiopacity of the radiopaque marker depends on the size of the marker material, the at least one structure cannot be arbitrarily small or large. The inventors have found that a diameter between 20 µm and 1000 µm is optimal since radiopacity is sufficient and the mechanical properties of the scaffold are not adversely affected due to large radiopaque markers. Therefore, in a preferred embodiment, the vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the at least one structure is adapted to hold a radiopaque marker having a diameter between 20 µm and 1000 µm.

Preferably, the at least one structure is adapted to hold a radiopaque marker having a diameter between 50 µm and 1200 µm, more preferably between 50 µm and 1100 µm, more preferably between 50 µm and 1000 µm, more preferably between 100 µm and 1000 µm, more preferably between 150 µm and 900 µm, and most preferably between 200 µm and 800 µm.

Thus, a preferred embodiment is directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and the at least one structure is adapted to hold a radiopaque marker having a diameter between 200 µm and 800 µm.

In other words, a preferred embodiment is directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and the radiopaque marker has a diameter between 200 µm and 800 µm.

Preferably, the at least one structure is adapted to hold a radiopaque marker having a thickness between 20 µm and 1000 µm, more preferably between 30 µm and 900 µm, more preferably between 35 µm and 800 µm, more preferably between 40 µm and 600 µm, and most preferably between 50 µm and 500 µm.

Thus, a preferred embodiment is directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and the at least one structure is adapted to hold a radiopaque marker having a thickness between 50 µm and 500 µm.

In other words, a preferred embodiment is directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium and wherein the vascular scaffold is completely covered with an inorganic coating comprising magnesium fluoride., and the radiopaque marker has a thickness between 50 µm and 500 µm.

In a further embodiment, the vascular scaffold made of a biodegradable magnesium alloy comprises a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and the at least one structure is adapted to hold a radiopaque marker having a diameter between 20 µm and 1000 µm and having a thickness between 50 µm and 500 µm.

The inventors have found that most rare-earth metals (Group 3 elements and lanthanides) are not suitable as radiopaque markers despite their high density and atom number (see Example 4). Some rare-earth metals are not compatible with hydrofluoric acid passivation. Tantalum as well as neodymium dissolves in hydrofluoric acid. Other rare-earth metals lose their form-fit or force-fit connection with the scaffold structure. Gadolinium increases in volume during passivation, whereby the form-fit or force-fit connection with the structure is lost and a secure hold in the structure can no longer be guaranteed. Passivation of yttrium did not lead to a coating covering the marker completely. In addition yttrium has a low mass attenuation coefficient.

Thus, the inventors have identified dysprosium as suitable radiopaque marker material for the inventive vascular scaffolds described herein. Scaffolds with dysprosium marker could be fluoridated without residue release of particle formation. The dysprosium marker showed no observable volume increase after fluoridation. The dysprosium marker sits tight in the eyelet before and after fluoridation. No loosening or release of the marker occurred (see Example 4).

More preferably, the radiopaque marker is pure dysprosium. Thus, in a preferred embodiment, the vascular scaffold made of a biodegradable magnesium alloy comprises a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is pure dysprosium, wherein the vascular scaffold is completely covered with an inorganic coating comprising magnesium fluoride.

"**Pure** rare-earth metal", as used herein, refers to a rare-earth metal material having a purity of at least 95%, preferably at least 96%, preferably at least 97%, preferably at least 98%, preferably at least 98.5%, preferably at least 99.0%, preferably at least 99.5%, preferably at least 99.6%, preferably at least 99.7%, preferably at least 99.8%, preferably at least 99.9%, and most preferably at least 99.99%.

The inventive vascular scaffold is completely coated with an **inorganic coating comprising magnesium fluoride.** The inorganic coating improves the corrosion behaviour of the vascular scaffold and lowers crack formation at small layer thicknesses of few micrometers and therefore prevents early degradation of the scaffold.

The inventors have found that a complete coating of the scaffold and the radiopaque marker is required to prevent the cylindrical body from galvanic corrosion (see Example 2). An organic coating, such as a PLLA coating cannot prevent the galvanic corrosion. Instead strong local corrosion is observed underneath the coating, as water and ions diffuse quickly through the polymer coating, triggering galvanic corrosion. This leads to the disposed marker degrading much faster than the rest of the scaffold, causing the markers to fall out and impairing the radial support force of the scaffold (see Example 3).

The inorganic magnesium fluoride coating of the scaffold can be produced by transformation of the base material on the surface area/edge zone area or as an applied substrate. Thereby, the magnesium fluoride coating of the scaffold by transformation of the base material on the surface area/edge zone area means the formation of an inorganic layer of the scaffold material itself. For transformation of the base material on the surface area/edge zone area, ion implantation or chemical transformation such as reaction with hydrofluoric acid, potassium fluoride, etc. can be used. For production of an inorganic coating of a scaffold as an applied substrate, the coating comprises magnesium fluoride, which can be applied by using the various chemical and physical methods as described herein.

The application of the inorganic coating is not limited to the outer surface of a scaffold but can also be generated within the scaffold if required. Depending on the test parameters used, an ion-implanted edge zone can be created by using an ion implantation process. The layer thickness of the edge zone can be modified by the choice of the experimental parameters.

The inorganic coating or the surface modification by ion implantation of the vascular scaffolds according to the invention preferably affects the struts of the cylindrical scaffold body. The scaffold can be coated on both sides the abluminal as well as the luminal side of the scaffold body or only on one side. The coatings on the luminal or abluminal side of the scaffold may also be different, e.g. in the amount of active agent or in the composition of the organic compounds.

Surprisingly, it has been found that the vascular scaffold according to the invention can be coated with the inorganic coating comprising magnesium fluoride without corrosion and/or abrasion of the radiopaque marker. In other words, the inventive vascular scaffold can be coated together with the radiopaque marker such that the inorganic coating covers the vascular scaffold including the cylindrical scaffold body and the radiopaque marker.

In case the inorganic coating is applied by dipping the vascular scaffold in hydrofluoric acid (passivation), the cylindrical scaffold body is covered with a coating comprising magnesium fluoride and the radiopaque marker is covered with a coating comprising a rare-earth metal fluoride. Thus, one embodiment is directed to a vascular scaffold made of a biodegradable magnesium alloy comprising a cylindrical scaffold body formed of interconnected struts, wherein the interconnected struts comprise at least one structure designed to hold a radiopaque marker wherein the at least one structure holds a rare-earth metal as radiopaque marker in a form-fitting or force-fitting manner, and wherein the cylindrical scaffold body is covered with a coating comprising magnesium fluoride and the radiopaque marker is covered with a coating comprising a rare-earth metal fluoride.

Preferably, the layer thickness of the inorganic coating is between 0.01 µm to 100 µm, more preferably between 0.02 µm and 90 µm, more preferably between 0.03 µm and 80 µm, more preferably between 0.05 µm and 70 µm, more preferably between 0.06 µm and 60 µm, more preferably between 0.07 µm and 50 µm, more preferably between 0.08 µm and 40 µm, more preferably between 0.09 µm and 20 µm, and most preferably between 0.1 µm and 10 µm.

The inorganic coating may comprise further inorganic as well as organic materials in addition to magnesium fluoride and rare-earth metal fluoride. Inorganic materials traditionally include the elements and all compounds that do not contain carbon. Additionally, there are several exceptions of carbon compounds, which are structured exactly like typical inorganic materials or are historically allocated to the field of inorganic chemistry. These include the hydrogen-free chalcogenides of carbon, carbonic acid and carbonates, carbides as well as ionic cyanides, cyanates and thiocyanates. An inorganic coating as used herein refers to a layer applied on the struts of a scaffold, wherein the layer consists of magnesium fluoride and one of these inorganic compounds, mixtures thereof or contains at least magnesium fluoride as main component, i.e. at least 50%, preferably at least 80% and even more preferably at least 90%. A layer of an antirestenotic active agent or an organic layer of one or more organic compounds, preferably organic polymers, may be applied to the inorganic layer, wherein this organic layer may contain this antirestenotic active agent as well, so that no third active agent layer has to be applied. Thus, the layer of one or more organic compounds may contain one or more antirestenotic active agent(s) or the antirestenotic active agent may be applied to the layer of one or more organic compounds, wherein the in this case middle organic layer may contain no, the same or another or more than one antirestenotic active agents.

It may be advantageous that the abluminal coating (facing the vessel wall) dissolves more slowly than the luminal scaffold coating (facing the vessel lumen). In addition, a scaffold is preferred which only has micropores, holes, openings or channels in the luminal, inorganic, biologically degradable coating. Hydrogen gas is formed during degradation of magnesium alloys. This is one reason why it is preferred that the lumen and blood flow side of the inorganic biodegradable coating, but the abluminal side of the coating not as in this case the gas is washed away and distributed with the blood flow and cannot accumulate between the scaffold and the vessel wall.

If not generated by the coating process itself, these micropores, holes, openings and/or channels may be mechanically, chemically, thermally or optically introduced in the inorganic coating after it has been applied to the scaffold. For example, this may done by mechanical treatment such as sandblasting, by chemical processes such as etching, by mechano-chemical processes such as polishing, by thermal processes such as melting or baking, or by optical processes such as laser treatment.

In one embodiment, the inorganic coating is designed in such a way that the scaffold body can dissolve in the coating and that both hydrogen gas and the metal ions are mainly released into the blood on the luminal side of the coating, but not directly leaking into the surrounding tissue.

However, a vascular scaffold according to the invention, wherein the inorganic coating has no micropores, holes, openings or channels is preferred. This applies in particular to inorganic coatings without an active agent.

It is preferred that the scaffold body of biodegradable magnesium alloy is degraded under physiological condition before the inorganic coating is degraded, so that after degradation of the scaffold body an empty shell, ingrown in the vessel wall, remains, which however is flexible and does not longer exert any significant pressure on the vessel wall and even adapts well to the new course of the vessel. After complete dissolution of the scaffold body, the inorganic coating may also be degraded biologically, so that the scaffold is fully dissolved after several months. Thereby, the degradation of the inorganic coating should be uniform and without the risk of detaching fragments.

### Biodegradable magnesium alloy

The cylindrical scaffold body of the vascular scaffold according to the invention consists of a biodegradable magnesium alloy. This alloy consists preferably of 0.1 to 15.5% by wt. Dy and 0.01 to 1.5% by wt. Nd or 0.01 to 1.5% by wt. Eu or 0.01 to 1.5% by wt. Nd and Eu together, 0.0% by wt. - 2.0% by wt. zinc and 0.0% by wt. - 1.0% by wt. zirconium, the remainder being up to 100% by weight of Mg. This means that these alloys contain preferably 80.0% by wt. to 99.89% by wt. magnesium and more preferably 80.0% by wt. to 97.0% by wt. magnesium and further preferably 85.0% by wt. to 95.0% by wt. magnesium. This alloy may also contain other metals and unavoidable impurities. Preferred ranges for the components Dy, Nd, Eu, Zn and Zr are described in detail below.

With other words, the magnesium alloy, of which the cylindrical scaffold body consists, comprises preferably

| | | |
|---|---|---|
| 0.1% by wt. to | 15.5% by wt. | Dy and |
| 0.01% by wt. to | 1.5% by wt. | Nd or |
| 0.01 % by wt. to | 1.5% by wt. | Eu or |
| 0.01 % by wt. to | 1.5% by wt. | Nd and Eu together |
| 0.0% by wt. to | 2.0% by wt. | zinc and |
| 0.0% by wt. to | 1.0% by wt. | zirconium and |
| at least | 80.0% by wt. | magnesium. |

The cylindrical scaffold body of the scaffold is preferably made of magnesium alloys comprising 0.1 to 15.5% by wt. Dy and 0.01 to 1.5% by wt. Nd or 0.01 to 1.5% by wt. Eu or 0.01 to 1.5% by wt. Nd and Eu together, 0.0% by wt. - 2.0% by wt., preferably to 1.5% by wt. zinc and further comprising 0.1% by wt. - 0.75% by wt. zirconium. Also these alloys may further contain unavoidable impurities.

Particularly preferred is if the cylindrical scaffold body of the scaffold according to the invention consists of magnesium alloy, which in relation to the total weight of the alloy (in % by wt.) contains following components:

| | |
|---|---|
| 81.25% by wt. - 99.89% by wt. | magnesium |
| 0.1% by wt. - 15.0% by wt. | dysprosium |
| 0.01% by wt. - 1.5% by wt. | neodymium and/or europium |
| 0.0% by wt. - 1.5% by wt. | zinc |
| 0.0% by wt. - 0.75% by wt. | zirconium |

wherein the stent has an inorganic coating comprising magnesium fluoride and an organic coating.

Magnesium (Mg) as main component of the alloy has been chosen as Mg is biologically degradable and an essential element of the body which does not accumulate in the body in a way that it is harmful. Excessive magnesium is generally excreted naturally. The magnesium alloys of the inventive scaffold consist preferably of at least 80% by wt. of magnesium.

For the production of scaffolds, especially strength characteristics and the corrosion behavior have been taken into consideration to provide an alloy being as strong and corrosion-resistant as possible, wherein the control of the corrosion behavior can be adjusted via additional coatings, such as an inorganic coating and an organic coating.

It has been found that the minimum level of corrosion of the magnesium alloys, as described herein, occurs at a content of 10% by weight Dy. Thus, it is especially preferred if the content of dysprosium in the respective alloys is approximately 10% by weight ± 2% by weight. The corrosion is the crucial property for the degradation rate of the scaffold in the vessel. It is important that a scaffold does not lose its stability too early, so that no fragments are detached, and the stability is ensured by the scaffold until it can be reapplied by the vessel alone and until the scaffold has grown into the vessel wall. An additional inorganic coating and an additional organic coating may serve in particular to adjust the resorption kinetics and to control the degradation rate of the scaffold, so that the scaffold is not dissolved before it has grown into the vessel wall. Furthermore, dysprosium together with magnesium form intermetallic precipitates. The high solubility of dysprosium in magnesium ensures that the required heat treatments in the production of stents can be performed successfully, that the precipitates are dissolved and precisely precipitated again and that properties such as strength, ductility and corrosion behavior can be adjusted over a wide range in this way.

Neodymium and europium have also shown no negative effects on cells *in vitro.* Europium was even better tolerated than neodymium. Both elements are practically insoluble in magnesium and form intermetallic phases with magnesium, which are also not dissolved by the heat treatments required for the production of scaffolds. These precipitates are localized on the grain boundaries and stabilize these so that the fine grain of the forming is retained. It has been shown that 1% by weight of neodymium or 1% by weight of europium or 1% by weight of europium and neodymium together are sufficient.

Zinc improves the casting characteristics of the magnesium alloy and has a strengthening effect. Thus, the fatigue behavior and tensile strength can be increased by addition of zinc up to 3% by weight. The tensile strength should preferably be as high as possible and preferably more than 180 MPa (≥ 180 MPa), further preferred more than 200 MPa (≥ 200 MPa). However, the tendency for hot crack formation increases with more than 1% by weight Zn. Thereby, micropores are formed, which negatively affect tensile strength and ductility of an alloy. They act as inner notches so that in tensile tests a material fails generally clearly below the maximal achievable strength at a fractional amount of the theoretical ductility. In general, a disadvantageous effect on the processing behavior and the mechanical properties of the alloys described herein is shown with more than 2% by weight zinc. Zinc is an essential element for human beings, which is part of many enzymes and has many functions. Among others, zinc has an anti-inflammatory effect. Nevertheless, with high doses acute poisoning can occur and a long-term supply causes disorders, especially of the iron and copper metabolism (cf. Guidelines for drinking-water quality, World Health Organization, 1996). Therefore, toxic side effects cannot be excluded at a content of 4% by weight zinc and more. Thus, the amount of zinc should be below 2.0% by weight, preferred below 1.8% by weight, more preferred below 1.6% by weight, even more preferred below 1.4% by weight and especially preferred below 1.2% by weight. In several embodiments of the present invention the magnesium alloys of the inventive scaffold are free of zinc.

Zirconium (Zr) may be present in the magnesium alloy in addition to zinc or also instead of zinc. Here, Zr is used as grain refiner. For a magnesium alloy one can alloy Zr in a range up to approximately 0.75% by weight for the production of the inventive scaffolds. Also larger amounts of Zr of 2% by weight or also 3% by weight result in a similar well grain refinement, but increase the price of the alloy remarkably and, in addition, lead to an embrittlement of the alloy, which in turn leads to a decrease of the ductility. Since, in regard to grain refinement, the results achieved with significantly smaller quantities of Zr of 0.0% by weight to 0.50% by weight were as good as with 1% by weight, 2% by weight or 3% by weight and in addition with an amount of Zr below 0.75% by weight no embrittlement occurs, 0.0% by weight to 0.1% by weight and further preferred 0.1% by weight to 0.75% by weight zirconium are used according to the invention.

The influence of Zr has been exemplarily examined with the magnesium alloy containing 10% by weight Dy and 1% by weight Nd. Permanent mold direct chill casting ("*Tütengussverfahren*") has been used as production process. For materials produced by permanent mold direct chill casting, one can assume that a cast part shows a homogenous microstructure and that the alloying elements are homogenously distributed too. However, the structure is comparatively rough and the grain size is in the range of several millimeters.

Therefore, it is preferred, if an alloy has further 0.02 - 0.80% by weight, preferred 0.04 - 0.60% by weight, preferred 0.05 - 0.55% by weight, further preferred 0.06 - 0.50% by weight, even more preferred 0.07 - 0.45% by weight, even more preferred 0.08 - 0.40% by weight, even more preferred 0.09 - 0.35% by weight, even more preferred 0.10 - 0.30% by weight, even more preferred 0.12 - 0.28% by weight and especially preferred 0.15 - 0.25% by weight zirconium.

The present invention further relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises dysprosium.

The present invention further relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the cylindrical scaffold body contains the following components based on the total weight of the alloy (given in % per wt.):

| | | |
|---|---|---|
| 80% | by wt. - 94.9% by wt. | magnesium |
| 5.0% | by wt. - 13.0% by wt. | dysprosium |
| 0.1% | by wt. - 2.0% by wt. | neodymium |
| 0.0% | by wt. - 2.0% by wt. | zinc |
| 0.0% | by wt. - 3.0% by wt. | zirconium. |

Optionally, the amount of neodymium in this alloy may be substituted by europium, or one may add further 0.1% by wt. - 2.0% by wt. europium.

It goes without saying that all components of an alloy must add up to 100% by wt. Unless not explicitly mentioned, the herein disclosed alloys may contain unavoidable impurities, which are in the range of the lower detection limit or in the range of 1 ppm up to 0.4% by wt., preferably up to 0.3% by wt., further preferably up to 0.2% by wt., and in particular preferably up to 0.1% by wt.. Silicon as main ingredient of the impurities may reach already 0.3% by weight. Silicon (Si) should not be present in an amount over 0.4% by wt., preferably over 0.3% by wt., and more preferably over 0.2% by wt., in the alloy. It is therefore especially preferred if the unavoidable impurities except silicon represent in total less than 0.3% by weight, preferred less than 0.2% by weight, further preferred less than 0.1% by weight, further preferred less than 0.05% by weight and especially preferred less than 300 ppm. These impurities (including silicon) may also be present in the alloy, if they are not explicitly listed as an alloying element and, in the case not being mentioned, are added to the weight proportion of that component of the alloy, through which they have been introduced into the alloy.

In another embodiment, the vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy is composed of the following components based on the total weight of the alloy:

| | |
|---|---|
| 80.0% by wt. - 99.9% by wt. | magnesium |
| 0.1% by wt. - 13.0% by wt. | dysprosium |
| 0.0% by wt. - 3.0% by wt. | neodymium |
| 0.0% by wt. - 1.0% by wt. | zinc |
| 0.0% by wt. - 1.0% by wt. | zirconium |
| 0.0% by wt. - 2.0% by wt. | other metals, metal salts and non metals, which are commonly referred to as impurities. |

It is preferred, when the alloy has 0.1 - 20.0% by wt., preferably 0.15 - 19.0% by wt., preferably 0.16 - 18.0% by wt., preferably 0.17 - 17.0% by wt., preferably 0.18 - 17.0% by wt., preferably 0.19 - 16.5% by wt., preferably 2.0 - 16.0% by wt., preferably 2.1 - 16.0% by wt., preferably 2.2 - 15.0% by wt., preferably 2.2 - 14.0% by wt., preferably 2.3 - 13.0% by wt., preferably 2.4 - 13.5% by wt., preferably 2.5 - 13.0% by wt., preferably 2.6 - 12.5% by wt., preferably 2.7 - 12.7% by wt., preferably 2.8 - 12.4% by wt., preferably 2.9 - 12.0% by wt., preferably 3.0 - 12.2% by wt., preferably 3.1- 12.0% by wt., preferably 3.2 - 11.5% by wt., preferably 3.3 - 11.5% by wt., preferably 3.4 - 11.0% by wt., and more preferred 0.35 - 11.0% by wt. dysprosium.

Preferably, the amount of neodymium is in the range of 0.0 - 8.0% by wt., more preferably from 0.1 - 5.0% by wt., still more preferably 0.2 - 4.0% by wt., even more preferably 0.3 - 3.5% by wt. and especially preferably of 0.5 to 3.2% by wt.

Together with neodymium (Nd) or instead of Nd also europium (Eu) in the alloy in proportions from 0.0 - 8.0% by wt., more preferably 0.1 - 5.0% by wt., even more preferably 0.2 - 4.0% by wt., still more preferably 0.3 - 3.5% by wt., especially preferably from 0.5 - 3.2% by wt.

It is further preferred that the sum of the weight proportions of Nd and Eu in the alloy is from 0.01 - 8.0% by wt., more preferably from 0.1 to 5.0% by wt., still more preferably 0.2 - 4.0% by wt., and especially preferably from 0.5 - 3.2% by wt.

The sum of the weight proportions of dysprosium and neodymium is preferably in the range of 1.1 - 18.0% by wt., more preferably between 1.6 to 15.5% by wt., even more preferably, and particularly preferably from 2.1 to 13.0% by wt.

It is further preferred that the alloy has furthermore 0.0 - 4.0% by wt., more preferably 0.0 - 3.0% by wt., still more preferably 0.0 - 2.0% by wt., even more preferably 0.1 - 1.5% by wt. and especially preferably 0.2 - 1.3% by wt. zinc (Zn).

In addition to the aforementioned components a magnesium alloy from which the cylindrical scaffold body of the inventive vascular scaffold was made may also contain 0.0% by wt. - 5.0% by wt., preferably 0.1% by wt. - 4.0% by wt., more preferably 0.2% by wt. - 3.0% by wt. and especially preferred in total not more than 2.5% by wt. other metals, metal salts, non-metals, carbon, sulphur, silicon, nitrogen, oxygen, and/or hydrogen.

Furthermore, it is preferred that the elements beryllium, aluminium and manganese are each present below 300 ppm, preferably below 200 ppm and more preferably below 150 ppm in the magnesium alloys from which the body of the inventive vascular scaffold was made.

The maximum amount of 2.5% by wt. impurities includes the other metals or non-metals such as for example silicon, carbon, oxygen, nitrogen, hydrogen or sulphur, also if they are additionally explicitly listed. The term "impurities" as used herein refers to all alloy components except of magnesium, dysprosium, neodymium, europium, zinc and zirconium irrespective of explicitly mentioning these or not.

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.5% by wt. | magnesium |
| 0.1% by wt. - 13.0% by wt. | dysprosium |
| 0.2% by wt. - 4.0% by wt. | neodymium |
| 0.2% by wt. - 3.0% by wt. | zinc. |

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | | |
|---|---|---|
| 88% by wt. | | magnesium |
| 1 0% by wt. | . | dysprosium |
| 1 % by wt. | . | neodymium |
| 1 % by wt. | . | zinc. |

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.65% by wt. | magnesium |
| 0.1% by wt. - 14.0% by wt. | dysprosium |
| 0.2% by wt. - 4.0% by wt. | neodymium |
| 0.05% by wt. - 2.0% by wt. | zirconium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | | |
|---|---|---|
| 88% by wt. | | magnesium |
| 1 0% by wt. | . | dysprosium |
| 1 % by wt. | . | neodymium |
| 1 % by wt. | . | zirconium. |

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.45% by wt. | magnesium |
| 0.1% by wt. - 13.0% by wt. | dysprosium |
| 0.2% by wt. - 4.0% by wt. | neodymium |
| 0.2% by wt. - 2.0% by wt. | zinc |
| 0.05% by wt. - 1.0% by wt. | zirconium. |

A particularly preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 87% by wt. | magnesium |
| 1 0% by wt. | dysprosium |
| 1 % by wt. | neodymium |
| 1 % by wt. | zinc |
| 1 % by wt. | zirconium. |

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.4% by wt. | magnesium |
| 0.1% by wt. - 13.0% by wt. | dysprosium |
| 0.2% by wt. - 3.5% by wt. | neodymium |
| 0.2% by wt. - 1.0% by wt. | zinc |
| 0.1% by wt. - 2.5% by wt. | impurities such as other metals, metal salts and non metals. |

A preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.6% by wt. | magnesium |
| 0.1% by wt. - 13.0% by wt. | dysprosium |
| 0.1% by wt. - 3.0% by wt. | neodymium |
| 0.1% by wt. - 2.0% by wt. | zinc |
| 0.1% by wt. - 2.0% by wt. | impurities such as other metals, metal salts and non metals. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.55% by wt. - 99.6% by wt. | magnesium |
| 0.1% by wt. - 13.0% by wt. | dysprosium |
| 0.1% by wt. - 3.0% by wt. | neodymium |
| 0.1% by wt. - 0.75% by wt. | zirconium |
| 0.1% by wt. - 2.0% by wt. | impurities such as other metals, metal salts and non metals. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.55% by wt. - 99.5% by wt. | magnesium |
| 0.1% by wt. - 11.0% by wt. | dysprosium |
| 0.1% by wt. - 3.2% by wt. | neodymium |
| 0.1% by wt. - 2.0% by wt. | zinc |
| 0.1% by wt. - 0.75% by wt. | zirconium |
| 0.1% by wt. - 2.5% by wt. | impurities such as other metals, metal salts and non metals. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.25% by wt. - 94.6% by wt. | magnesium |
| 5.0% by wt. - 12.5% by wt. | dysprosium |
| 0.1% by wt. - 2.0% by wt. | europium |
| 0.1% by wt. - 2.0% by wt. | zinc |
| 0.1% by wt. - 0.75% by wt. | zirconium |
| 0.1% by wt. - 2.5% by wt. | impurities such as other metals, metal salts and non metals. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.4% by wt. | magnesium |
| 0.1% by wt. - 11.0% by wt. | dysprosium |
| 0.1% by wt. - 2.0% by wt. | europium |
| 0.1% by wt. - 3.0% by wt. | neodymium |
| 0.1% by wt. - 2.0% by wt. | zinc |
| 0.1% by wt. - 1.0% by wt. | zirconium |
| 0.1% by wt. - 1.0% by wt. | impurities such as other metals, metal salts and non metals. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.65% by wt. | magnesium |
| 0.1% by wt. - 15.0% by wt. | dysprosium |
| 0.2% by wt. - 3.0% by wt. | europium |
| 0.05% by wt. - 2.0% by wt. | zirconium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 88% by wt. | magnesium |
| 1 0% by wt. | dysprosium |
| 1 % by wt. | europium |
| 1 % by wt. | zirconium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.75% by wt. | magnesium |
| 0.1% by wt. - 15.0% by wt. | dysprosium |
| 0.2% by wt. - 4.0% by wt. | europium |
| 0.05% by wt. - 4.0% by wt. | zinc. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 88% by wt. | magnesium |
| 1 0% by wt. | dysprosium |
| 1 % by wt. | europium |
| 1 % by wt. | zinc. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 80.0% by wt. - 99.6% by wt. | magnesium |
| 0.1% by wt. - 14.0% by wt. | dysprosium |
| 0.2% by wt. - 2.0% by wt. | europium |
| 0.05% by wt. - 2.0% by wt. | zinc |
| 0.05% by wt. - 2.0% by wt. | zirconium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 87% by wt. | magnesium |
| 1 0% by wt. | dysprosium |
| 1 % by wt. | europium |
| 1 % by wt. | zinc |
| 1 % by wt. | zirconium. |

A particularly preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 87.8% by wt. | magnesium |
| 10% by wt. | dysprosium |
| 1.0% by wt. | europium |
| 1.0% by wt. | zinc |
| 0.2% by wt. | zirconium. |

A further particularly preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 86.8% by wt. | magnesium |
| 10% by wt. | dysprosium |
| 1.0% by wt. | europium |
| 1.0% by wt. | zinc |
| 0.2% by wt. | zirconium. |

A further particularly preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 87.8% by wt. | magnesium |
| 10% by wt. | dysprosium |
| 1.0% by wt. | neodymium |
| 1.0% by wt. | europium |
| 1.0% by wt. | zinc |
| 0.2% by wt. | zirconium. |

All percentages by weight specified in this disclosure refer to the total weight of the corresponding alloy. Thus it applies to all compositions, mentioned herein, that the sum of all components in total must add up to 100.00% by wt.. That means, after addition of all listed components of the magnesium alloy, the difference to 100% by wt. is magnesium as the main component.

Furthermore, the present invention comprises preferably vascular scaffolds, which cylindrical scaffold body consists of biodegradable magnesium alloys, which comprise yttrium (Y), gadolinium (Gd), calcium (Ca), manganese (Mn), cerium (Ce), scandium (Sc), Indium (In), lithium (Li) or erbium (Er) as further components besides 80% by wt. magnesium, dysprosium, neodymium, europium, zinc, zirconium, and unavoidable, production-related impurities. That is, it is preferred, if the components of the alloy, besides the magnesium as basis, are selected from the following group consisting or comprising of: dysprosium, neodymium, europium, zinc, zirconium, yttrium, gadolinium, erbium, calcium, manganese, cerium, scandium, indium, lithium and unavoidable, production-related impurities. In several embodiments of the present invention the magnesium alloys of the stents are free of yttrium.

Consequently, a further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 93.0% by wt. - 94.0% by wt. | magnesium |
| 0.3% by wt. - 0.4% by wt. | dysprosium |
| 2.0% by wt. - 2.1% by wt. | neodymium |
| 0.3% by wt. - 0.5% by wt. | gadolinium |
| 0.3% by wt. - 0.4% by wt. | zirconium |
| 3.4% by wt. - 3.8% by wt. | yttrium |
| 0.0% by wt. - 0.02% by wt. | erbium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 92.0% by wt. - 93.0% by wt. | magnesium |
| 0.9% by wt. - 1.1% by wt. | dysprosium and gadolinium together |
| 2.1% by wt. - 2.3% by wt. | neodymium |

| | |
|---|---|
| 0.4% by wt. - 0.6% by wt. | zirconium |
| 3.9% by wt. - 4.3% by wt. | yttrium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 91.0% by wt. - 92.0% by wt. | magnesium |
| 0.7% by wt. - 0.8% by wt. | dysprosium |
| 0.6% by wt. - 0.8% by wt. | gadolinium |
| 1.9% by wt. - 2.1% by wt. | neodymium |
| 0.6% by wt. - 0.8% by wt. | zirconium |
| 3.9% by wt. - 4.2% by wt. | yttrium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 93.7% by wt. - 96.2% by wt. | magnesium |
| 1.0% by wt. - 1.5% by wt. | gadolinium |
| 2.0% by wt. - 3.1% by wt. | neodymium |
| 0.5% by wt. - 0.7% by wt. | zirconium |
| 0.1% by wt. - 0.5% by wt. | calcium |
| 0.2% by wt. - 0.5% by wt. | zinc. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 96.9% by wt. | magnesium |
| 2.5% by wt. | neodymium |
| 0.4% by wt. | zirconium |
| 0.2% by wt. | zinc. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 97.45% by wt. | magnesium |
| 0.75% by wt. | neodymium |
| 1.80% by wt. | manganese. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 97.45% by wt. | magnesium |
| 0.75% by wt. | cerium |
| 1.80% by wt. | manganese. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 90.0% by wt. | magnesium |
| 3.0% by wt. | gadolinium |
| 2.4% by wt. | yttrium |
| 0.4% by wt. | zirconium |
| 4.2% by wt. | scandium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 90.0% by wt. | magnesium |
| 3.0% by wt. | neodymium |
| 2.4% by wt. | yttrium |
| 0.4% by wt. | zirconium |
| 5.2% by wt. | scandium |
| 2.0% by wt. | indium. |

A further preferred embodiment of the invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the biodegradable magnesium alloy comprises or consists of the following components:

| | |
|---|---|
| 96.0% by wt. | magnesium |
| 4.0% by wt. | lithium. |

The vascular scaffold according to the invention is preferably a scaffold for blood vessels, urinary tracts, respiratory tracts, biliary tracts or the digestive tract. In turn, among these scaffolds the vascular implants or scaffolds for blood vessels or more generally for the cardiovascular system are preferred.

The herein disclosed magnesium alloys are selected in such a way that they are particularly suitable for production of resorbable or degradable endoprostheses and particularly of vascular implants or scaffolds, respectively.

The terms "bioresorbable" or "biodegradable", as used herein, means that the scaffold slowly dissolves in a human or animal organism over a certain time and at some point, only its degradation products are present in the body in a dissolved form. At this point in time, solid components or fragments of the implant are not present anymore. The degradation products should be substantially harmless in physiological terms and lead to ions or molecules which occur in the organism anyway or can be degraded by the organism to harmless substances, or can be excreted.

This means that a scaffold is biodegradable if the scaffold exhibits a significant weight reduction or chemical transformation after being inserted into a patient. Weight reduction may occur, e.g. by dissolution of the material. Preferred chemical transformations are oxidation/reduction, hydrolysis, substitution, and/or addition. Erosion can be the result of a chemical interaction of the scaffold with the body (the body itself or body fluids). Preferably, erosion occurs to a desired extent in a time frame that ensures that the essential functions of the scaffold such as lumen support are maintained for as long as needed. Scaffolds of the biologically biodegradable magnesium alloys having inorganic coatings as disclosed herein are resorbed within a period of 8 to 50 weeks, preferably 10 to 30 weeks, under physiological conditions.

Scaffolds are hereby grid-shaped or net-shaped endoprostheses which are inserted into a hollow organ or a body cavity to keep it open. The cylindrical scaffold body of a scaffold, which is herein referred to metallic struts without coating and without radiopaque marker, is not a solid tube but a mesh. If, for example, the cylindrical scaffold body is considered, it is cut from a solid tube, e.g. by a laser, so that as thin as possible individual struts are formed, which are interconnected. The arrangement and shaping of the struts and nodal points is called scaffold design. In the sense of the present invention, all common scaffold geometries can be used as magnesium scaffold according to the invention.

In a further embodiment of the present invention, the vascular scaffold is coated with an **organic coating**. The organic coating is present on top of the inorganic coating, which is directly applied on the scaffold body and the radiopaque marker.

Thus, the present invention is further directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, wherein the vascular scaffold further comprises an organic coating.

The organic coating for all herein disclosed vascular scaffolds may comprise or consist of one or more biostable or biodegradable substances. Preferably, the organic coating consists or comprises one or more organic polymers. Therefore, in one embodiment, the present invention is further directed to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the scaffold further comprises an organic coating comprising or consisting of one or more substances of the following group:
polydopamine, polyvinyl pyrrolidone, glycerol, poly hydroxyethyl methacrylates, polyethylene glycol, polypropylene glycol, polyvinyl alcohol polydioxanone, polycaprolactone, polygluconate, poly(lactic acid) polyethylene oxide copolymer, modified cellulose, polyhydroxybutyrate, polyamino acids, polyphosphate esters, polyvalerolactones, poly-e-decalactones, polylactonic acid, poly(glycolic acid), polylactides, preferably poly(L-lactide), poly(D,L-lactide), and copolymers as well as blends such as poly(L-lactide-co-glycolide), poly(D,L-lactide-co-glycolide), poly(L-lactide-co-D,L-lactide), poly(L-lactide-co-trimethylene carbonate)] (PTMC), polyglycolides, copolymers of polylactides and polyglycolides, poly(e-caprolactone), polyhydroxybutyric acid, polyhydroxybutyrates, polyhydroxyvalerates, polyhydroxybutyrate-co-valerates, poly(1,4-dioxane-2,3-dione), poly(1,3-dioxane-2-one), poly(para-dioxanones), polyanhydrides, poly(maleic anhydrides), polyhydroxy methacrylates, fibrin, polycyanoacrylates, polycaprolactone dimethylacrylates, poly-b-maleic acid polycaprolactone butyl acrylates, multiblock polymers of oligocaprolactone diols and oligodioxanone diols, poly(ether ester) multiblock polymers of PEG and poly(butylene terephthalate), polypivalolactone, polyglycolic acid-trimethylene carbonates, polycaprolactone glycolides, poly(g-ethyl glutamate), poly(DTH iminocarbonate), poly(DTE-co-DT-carbonate), poly(bisphenol A iminocarbonate), poly(ortho ester), polyglycolic acid-trimethylene carbonate, poly(trimethylene carbonate) poly(imino carbonate), poly(N-vinylpyrrolidone), polyvinyl alcohols, polyesteramides, glycolized polyesters, polyphosphoesters, polyphosphazenes, poly[(p-carboxyphenoxy) propane], poly(hydroxypentanoic acid), polyanhydrides, poly(ethylene oxide)-poly(propylene oxide), soft polyurethanes, polyurethanes with amino acid residues in the backbone, polyether esters such as polyethylene oxide, polyalkene oxalates, polyorthoesters and their copolymers, lipids, waxes, oils, polyunsaturated fatty acids, eicosapentaenoic acid, timnodonic acid, docosahexaenoic acid, arachidonic acid, linoleic acid, α linolenic acid, γ-linolenic acid, carrageenans, fibrinogen, agar-agar, starch, collagen, protein-based polymers, polyamino acids, synthetic polyamino acids, Zein, polyhydroxyalkanoates, pectic acid, actinic acid, carboxymethyl sulfate, albumin, hyaluronic acid, chitosan and derivatives thereof, heparan sulfate and derivatives thereof, heparins, chondroitin sulfate, dextran, ß-cyclodextrins, copolymers with PEG and polypropylene glycol, gum arabic, guar, gelatin, collagen, collagen-N-hydroxysuccinimide, lipids, phospholipids, polyacrylic acid, polyacrylates, poly(methyl methacrylate), poly(butyl methacrylate), polyacrylamide, polyacrylonitrile, polyamides, polyetheramides, polyethylene amine, polyimides, polycarbonates, polycarbourethanes, polyvinyl ketones, polyvinyl halides, polyvinylidene halides, polyvinyl ethers, polyisobutylenes, polyvinyl aromatic compounds, polyvinyl esters, polyvinylpyrrolidones, polyoxymethylenes, polytetramethylene oxide, polyethylene, polypropylene, polytetrafluoroethylene, polyurethanes, polyetherurethanes, silicone polyetherurethanes, silicone polyurethanes, silicone polycarbonate urethanes, polyolefin elastomers, polyisobutylenes, fluorosilicones, carboxymethylchitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halides, cellulose ethers, cellulose triacetates, shellac, parylene (poly-para-xylylene), parylene N, parylene C and/or parylene D, and copolymers of the aforementioned polymers.

Preferred polymers for the organic coating are:
polyvinyl pyrrolidone, polyhydroxyethyl methacrylates, polyethylene glycole, polypropylene glycole, polyvinyl alcohol, polydioxanone, polycaprolactone, poly(lactic acid)-polyethylene oxide-copolymer, polyvalerolactones, poly-ε-decalactones, polylactonic acid, polyglycolic acid, polylactides, preferably poly(L-lactide), poly(D,L-lactide), and copolymers as well as blends such as poly(L-lactide-co-glycolide), poly(D,L-lactide-co-glycolide), poly(L-lactide-co-D,L-lactide), poly(L-lactide-co-trimethylene carbonate) (PTMC), polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerates, poly(1,4-dioxane-2,3-dione), poly(1,3-dioxane-2-one), poly-para-dioxanones, polymaleic acid anhydrides, polyhydroxy methacrylates, fibrin, polycaprolactone dimethylacrylates, polycaprolactone butyl acrylates, polycaprolactone glycolides, poly(g-ethyl glutamate), polyorthoesters, polyvinyl alcohols, polyester amides, polyurethanes polyacrylates, polymethyl methacrylate, polybutyl methacrylate, polyacrylamide, polyamides, polyetheramides, polyethylene amine, polyimides, polycarbonates, polycarbourethanes, polyvinyl ketones, polyvinyl ethers, polyisobutylenes, polyvinyl aromatic compounds, polyvinyl esters, polyoxymethylenes, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, polyisobutylenes, fluorosilicones, polyaryletheretherketones, polyetheretherketones, polyethylene terephthalate, polyvalerates, polysulfones, polysiloxanes, polydimethylsiloxanes, poly-paraxylylenes (Parylenes) such as Parylene N, Parylene C and/or Parylene D, and copolymers and/or mixtures of the aforementioned polymers.

The organic coating of the vascular scaffolds according to the invention is preferably metal-free, i.e. it does not contain any metal-containing compounds such as sodium, aluminium, magnesium, iron, zircon or titanium. The organic coating preferably contains no organometallic compounds, metal alkoxides or polymeric metal alkoxides. In particular the organic coating should not consist of titanium ethylene glycol, titanium propylene glycol, zircon ethylene glycol, zircon propylene glycol, hafnium ethylene glycol, hafnium propylene glycol or polyaluminium ethylene glycol. Organometallic compounds are referred to as compounds having a metal-carbon bond.

Thus, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and the scaffold further comprises an organic coating which does not contain organometallic compounds, metal alkoxides or polymeric metal alkoxides.

Parylene is mostly the designation for completely linear, semi-crystalline, non-crosslinked aromatic polymers. The different polymers have different properties and can be divided into four basic types, namely parylene C, parylene D, parylene N and parylene F, the structure of which is shown in the following:

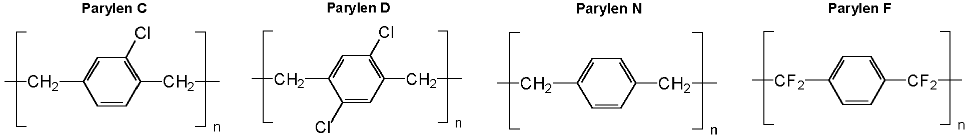

The simplest monomer in the parylene group is parylene N (poly-para-xylylene). There exist also two chlorinated polymers: parylene C (chloropoly-para-xylylene) and parylene D (di-chloro-poly-para-xylylene). In case of parylene F (poly(tetrafluoropara-xylylene)), the methylene units are fluorinated.

Parylene C has the lowest melting point of only 290 °C of the aforementioned parylenes, is characterized by good mechanical properties and corrosion resistance to corrosive gases as well as very low permeability to moisture. Parylene C is a biocompatible polymer and therefore suitable for use in physiological environments.

In a preferred embodiment of the present invention, the organic coating comprises one or more substances of the following group: poly-ε-caprolactone (PCL), poly(L-lactide-co-glycolide) (PLGA), poly(L-lactide) and parylene.

Therefore, the present invention relates to a vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, wherein the vascular scaffold is completely covered with an inorganic coating comprising magnesium fluoride, and wherein the scaffold further comprises an organic coating comprising one or more substances of the following group: poly(ε-caprolactone), poly(L-lactide-co-glycolide), poly(L-lactide) and parylene.

As organic coating the polymers poly-ε-caprolactone (PCL), poly(L-lactide-co-glycolide) (PLGA), poly(L-lactide) and parylene are also preferred, whereby the following polymers provided comparable results in preliminary tests:
polyvinyl pyrrolidone, polyhydroxyethyl methacrylates, polyhydroxybutyrate, polyvalerolactones, poly-ε-decalactones, poly(D,L-lactide-co-glycolide), polyglycolides, copolymers of polylactides and polyglycolides, polyhydroxyvalerates, polyhydroxy methacrylates, polycaprolactone dimethylacrylates, polycaprolactone butyl acrylates, polycaprolactone glycolides, polyurethanes, polymethyl methacrylate, polyvinyl ketones, polyvinyl ethers, polyvinyl aromatic compounds, polyvinyl esters, polyethylene terephthalate, polyvalerates, and polysulfones.

Preferably, the layer thickness of the organic coating is 0.01 µm to 100 µm.

In further embodiments, at least one antiinflammatory, antiproliferative, antiangiogenic, antirestenotic (anti-restenosis), antineoplastic, antimigrative and/or antithrombogenic active agent is present on the scaffold body, in or on an inorganic coating (if present), and in or below an organic coating (if present). This active agent may be contained in covalently bound form or in adhesive or ionically bound form in the inorganic coating or be applied as additional layer. Coated endoprothesis as well as scaffolds are thereby obtained, which have at least one active agent in the inorganic coating, or which have an additional layer containing the active agent on the inorganic coating. Preferably, the at least one antiinflammatory, antiproliferative, antiangiogenic, antirestenotic, antineoplastic, antimigrative and/or antithrombogenic active agent is applied in the form of an additional active agent releasing layer (drug release system) to the surface of the scaffold body.

In further preferred embodiments, at least one antirestenotic active agent such as an antiinflammatory, antiproliferative, antiangiogenic, antirestenotic, antineoplastic, antimigrative and/or antithrombogenic active agent is present in or on an outer polymeric organic coating. Suitable antirestenotic active agents were already explicitly mentioned above. Preferred are derivates of sirolimus ("limus" derivatives) as well as paclitaxel. Particularly preferred is sirolimus itself. The active agent can be contained in covalently bound form or in adhesive or ionically bound form in the polymeric organic coating or applied as additional layer. Coated endoprothesis as well as stents are thereby obtained, which have at least one active agent in the polymeric coating, or which have an additional layer containing the active agent on the polymeric coating. Preferably, the at least one antiinflammatory, antiproliferative, antiangiogenic, antirestenotic, antineoplastic, antimigrative and/or antithrombogenic active agent is applied in form of an additional active agent releasing layer (drug release system) to the surface of the polymeric organic coating of the stent. This active agent releasing layer may be a pure active agent layer or a carrier layer of the aforementioned carrier materials or matrix materials or as well of a further polymer.

The at least one used antiinflammatory, antiproliferative, antiangiogenic, antirestenotic, antineoplastic, antimigrative and/or antithrombogenic active agent is preferably selected from the group comprising or consisting of: abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics, antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, bombrestatin, boswellic acids and its derivatives, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, dunaimycin, epirubicin, epothilone A and B, erythromycin, estramustine, etoboside, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclophosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mitoxanthrone, mycophenolate mofetil, ß-lapachone, podophyllotoxin, podophyllic acid 2-ethyl hydrazide, molgramostim (rhuGM-CSF), peginterferon α-2b, lanograstim (r-HuG-CSF), macrogol, selectin (cytokine antagonist), cytokinin inhibitors, COX-2 inhibitor, angiopeptine, monoclonal antibodies inhibiting muscle cell proliferation, bFGF antagonists, probucol, prostaglandins, 1-hydroxy-11-methoxycanthin-6-one, scopolectin, NO donors, pentaerythritol tetranitrate and sydnonimines, S-nitroso derivatives, tamoxifen, staurosporine, ß-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and its derivatives, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF 1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, ß and γ, histamine antagonists, serotonin blockers, apoptosis inhibitors, apoptosis regulators, halofuginone, nifedipine, paracetamol, dexpanthenol, clopidogrel, acetylsalicylic acid derivatives, streptomycin, neomycin, framycetin, paromomycin, ribostamycin, kanamycin, amikacin, arbekacin, bekanamycin, dibekacin, spectinomycin, hygromycin b, paromomycinsulfate, netilmicin, sisomicin, isepamicin, verdamicin, astromicin, apramycin, geneticin, amoxicillin, ampicillin, bacampicillin, pivmecillinam, flucloxacillin, mezlocillin, piperacillin, azlocillin, temocillin, ticarcillin, amoxicillin, clavulanic acid, ampicillin, sulbactam, piperacillin, tazobactam, sulbactam, cefamandol, cefotiam, cefuroxim, cefmenoxim, cefodizim, cefoperazon, cefotaxim, ceftazidim, cefsulodin, ceftriaxon, cefepim, cefpirom, cefoxitin, cefotetan, cefalexin, cefuroxim axetil, cefixim, cefpodoxim, ceftibuten, imipenem, meropenem, ertapenem, doripenem, aztreonam, spiramycin, azithromycin, telithromycin, quinopristin, dalfopristin, clindamycin, tetracycline, doxycyclin, minocyclin, trimethoprim, sulfamethoxazol, sulfametrol, nitrofurantoin, lomefloxacin, norfloxacin, ciprofloxacin, ofloxacin, fleroxacin, levofloxacin, sparfloxacin, moxifloxacin, vancomycin, teicoplanin, linezolid, daptomycin, rifampicin, fusidic acid, fosfomycin, trometamol, chloramphenicol, metronidazol, colistin, mupirocin, bacitracin, neomycin, fluconazol, itraconazol, voriconazol, posaconazol, amphotericin B, 5- flucytosin, caspofungin, anidulafungin, tocopherol, tranilast, molsidomine, tea polyphenols, epicatechin gallate, epigallocatechin gallate, leflunomide, etanercept, sulfasalazine, etoposide, dicloxacylline, tetracycline, triamcinolone, mutamycin, procainimide, retinoic acid, quinidine, disopyrimide, flecainide, propafenone, sotolol, natural and synthetically produced steroids, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir, zidovudine, clotrimazole, flucytosine, griseofulvin, ketoconazole, miconazole, nystatin, terbinafine, antiprozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-beta-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, maquiroside A, marchantin A, maytansin, lycoridicin, margetine, pancratistatin, liriodenine, bisparthenolidine, oxoushinsunine, periplocoside A, ursolic acid, deoxypsorospermin, psychorubin, ricin A, sanguinarine, manwu wheat acid, methylsorbifolin, chromones of spathelia, stizophyllin, mansonine, strebloside, dihydrousambaraensine, hydroxyusambarine, strychnopentamine, strychnophylline, usambarine, usambarensine, liriodenine, oxoushinsunine, daphnoretin, lariciresinol, methoxylariciresinol, syringaresinol, sirolimus (rapamycin) and its derivatives such as biolimus A9, everolimus, myolimus, novolimus, pimecrolimus, ridaforolimus, deoxorapamycin, tacrolimus FK 506, temsirolimus and zotarolimus, somatostatin, tacrolimus, roxithromycin, troleandomycin, simvastatin, rosuvastatin, vinblastine, vincristine, vindesine, teniposide, vinorelbine, trofosfamide, treosulfan, tremozolomide, thiotepa, tretinoin, spiramycin, umbelliferone, desacetylvismione A, vismione A and B, zeorin. and sulfur-containing amino acids such as cystine as well as salts, hydrates, solvates, enantiomers, racemates, mixtures of enantiomers, mixtures of diastereomers, metabolites, prodrugs, and mixtures of the aforementioned active agents. The concentration per active agent is preferably in the range of 0.001-500 mg per cm² coated surface of the endoprosthesis. Especially preferred active agents according to the present invention are paclitaxel, rapamycin and their derivatives, such as 6-α-hydroxy-paclitaxel, baccatin or other taxoteres, biolimus A9, myolimus, novolimus, pimecrolimus, tacroliums, temsirolimus, zotarolimus, everolimus, ridaforolimus or further "limus"-derivatives, erythromycin, midecamycin, josamycin and triazolopyrimidines. Particularly preferred is sirolimus (rapamycin). Also preferred are paclitaxel and the "limus" derivatives biolimus A9, myolimus, novolimus, pimecrolimus, tacroliums, temsirolimus, zotarolimus, everolimus, ridaforolimus or further sirolimus derivatives. All of the aforementioned compounds are herein referred to as antirestenotic active agents for simplicity reasons.

According to a preferred embodiment, the vascular scaffold further comprises an organic coating containing at least one antiproliferative, antiphlogistic and/or antithrombogenic active agent.

In a particular preferred embodiment, the vascular scaffold coating consists of a first inorganic coating comprising or consisting of magnesium fluoride covered by a second organic coating containing at least one antirestenotic active agent such as an antiproliferative, antiphlogistic and/or antithrombogenic active agent.

Between the inorganic coating and the organic coating containing the active agent, an additional adhesion-promoting layer may also be applied. Alternatively, a compound to support adhesion may be contained in the organic coating containing the active agent.

Thus, in a preferred embodiment the vascular scaffold comprises of a scaffold body of one of the herein disclosed biodegradable magnesium alloys, an inorganic coating comprising magnesium fluoride and an organic coating, optionally having at least one active agent.

Furthermore, it is preferred that the organic coating preferably consists of an organic polymer such as a parylene and contains an antirestenotic active agent and/or a third coating of the antirestenotic active agent present on this organic coating.

It is also possible that the active agent is applied to the vascular scaffold after the inorganic coating has already been applied and before the organic coating is applied to the metallic basic scaffold and the active agent does not form an own layer, but rather penetrates into the already present inorganic coating. Then, it is preferred that the active agent is not penetrating the entire coating, but rather remains in the outer part and forms a concentration gradient, which decreases in direction to the basic scaffold.

The inorganic layer or coating of the vascular scaffold itself preferably contains a maximum of 10% polymers and is even more preferably polymer-free. A layer of at least one antirestenotic active agent or an organic coating, for example of a polymer or a carrier substance or a matrix substance, with or without at least one antirestenotic active agent may be optionally present on the preferably polymer-free inorganic coating. Suitable carrier or matrices are described herein.

However, if the at least one active agent or combination of active agents is applied to the inorganic coating of vascular scaffolds, further substances may be applied as pharmacologically acceptable carriers or as matrix in combination with the at least one active agent or the combination of active agents. These carrier or matrices are also referred to as organic coatings or organic layers.

Polymers as wells as low molecular substances may serve as pharmacologically acceptable carriers, such as for example lactose, starch, sodium carboxymethyl starch, sorbitol, sucrose, magnesium stearate, dicalcium phosphate, calcium sulfate, talcum, mannitol, ethyl alcohol, polyvinyl alcohols, polyvinyl pyrrolidone, gelatine, naturally occurring sugars, naturally occurring as well as synthetic gums such as acacia gum or guar gum, sodium alginate, sodium benzoate, sodium acetate, glycerides, isopropyl myristates and palmitates, citrates, such as tributyl and triethyl citrates and their acetyl derivatives, phthalates such as dimethyl phthalate or dibutyl phthalate, etc. benzoic acid benzyl ester, triacetine, 2-pyrrolidone, boric acid, magnesium aluminium silicates, naturally occurring carob gum, gum karaya, guar, tragacanth, agar, cellulose, cellulose derivatives such as methyl cellulose, sodium carboxymethyl cellulose, hydroxypropyl methyl cellulose, microcrystalline cellulose as well as alginates, PLLA, parylene, polysulfones, shellac, aluminas and bentonites, polyethylene glycol and also waxes such as for example beeswax, carnauba wax, candelilla wax and the like can be used. The matrix substance of the second layer can also be identical to one inorganic compound of the first layer or to the entire composition of the first layer. The additional carrier or matrix substances can be used in a weight ratio of up to 70% by wt., preferably to 50% by wt. based on the used active agent(s).

A preferred embodiment is a vascular scaffold of one of the herein disclosed magnesium alloys, an inorganic coating comprising magnesium fluoride and, on the inorganic coating, an organic coating of a parylene. Furthermore, it is preferred that the organic coating contains an antirestenotic active agent and/or that an antirestenotic agent is applied thereto, such as preferably paclitaxel, sirolimus, biolimus A9, myolimus, novolimus, pimecrolimus, tacrolimus, temsirolimus, zotarolimus, everolimus, redaforolimus, or another sirolimus derivate and particularly preferred sirolimus (rapamycin).

The organic coating can generally be applied to the inorganic coating already present on the magnesium alloy of the scaffold body, by means of known methods such as spraying, dipping, plasma, brushing, syringe or pipetting method. Therefore, the vascular scaffold according to the invention can be coated by spraying, pipetting, brushing, spraying, plasma or dipping, whereby the organic compound or mixtures of the compounds are dissolved in a solvent and this solution is applied to the implant. The solvent or solvent mixture is then removed by evaporation at room temperature or heating.

The coating of the vascular scaffolds according to the invention can be carried out both before and after crimping onto a catheter balloon. If the coating is at first applied after the stent has been attached to a catheter balloon, a dipping or spraying method is preferred.

The organic coating should be performed relatively uniformly. The organic coating can have a thickness of about 0.01 to 100 µm and preferably of 0.1 - 50 µm, further preferably of 0.2 - 20 µm and most preferably of 0.5 - 10 µm.

In case of parylene as an organic coating, the layer thickness may be lower and is in the range from 0.001 to 10 µm, preferably in the range from 0.01 to 5 µm and further preferably in the range from 0.05 to 1.0 µm.

Furthermore, it is preferred that the ratio of the layer thickness of the inorganic coating and the organic coating of the vascular scaffolds according to the invention is 500:1 to 1:1,000,000, more preferably 400:1 to 1:700,000, even more preferably 100:1 to 1:200,000 and most preferably 20:1 to 1:100,000.

It is also preferred that the ratio of the layer thickness of the inorganic coating and the organic coating of the vascular scaffolds according to the invention is 1:500,000 to 1:1,000, more preferably 1:100,000 to 1:5,000, even more preferably 1:50,000 to 1:10,000 (inorganic : organic).

The inorganic coating can generally be applied to the magnesium alloy of the scaffold body by means of known methods such as spraying, dipping, plasma, brushing, syringe or pipetting method. The vascular scaffold according to the invention can be coated by spraying, pipetting, brushing, injection, plasma or dipping, whereby the inorganic compound or the inorganic material or mixtures of the compounds are dissolved in a solvent and this solution is applied to the implant. The solvent or solvent mixture is then removed by evaporation at room temperature or heating.

The coating of the vascular scaffolds according to the invention can be carried out both before and after crimping onto a catheter balloon. If the coating is at first applied after the vascular scaffold has been attached to a catheter balloon, a dipping or spraying method is preferred.

Various methods known in the art can be used to apply an inorganic coating to a vascular scaffold. For example, suspensions of inorganic particles in water or organic solvents such as ethanol can be used to produce the inorganic coatings.

According to an aspect of the present disclosure, the application and drying of the inorganic coating may be repeated several times to form a multi-layer coating. Likewise, the application and drying of the organic coating can be repeated several times to form a multi-layer coating. Application in several layers facilitates the formation of the inorganic coating or organic coating with the desired thickness (e.g. in the order of µm). More active agent can be embedded in a thicker coating.

Ion implantation can also be used for inorganic coating of surface areas/edge areas of the vascular scaffold according to the invention. Ion implantation is a method of introducing foreign atoms in the form of ions into the base material by bombarding the alloy with accelerated ions in a high vacuum. The ions are initially generated by means of an ion source, extracted by an electric field and then separated by a mass separator according to their mass. The ions are then accelerated and directed onto the alloy with an electric field. The ions are implanted into the alloy. Among the most important parameters of ion implantation are the acceleration energy with which the ions are accelerated, the ion type and the implantation dose. The first two parameters determine the penetration depth of the ions into the alloy and the implantation dose determines the concentration of the implanted ions. With ion implantation, different ions may be incorporated into the alloy, e.g. fluoride ions, oxygen ions, carbon ions. Implantation can cause damage to the crystal lattice of the alloy depending on the mass of the implanted ions and the implantation dose. In such cases, the alloy needs to be subjected to a high-temperature process after an implantation step in which the foreign atoms are incorporated into the lattice. This process is also referred to as healing. The healing process can, for example, be accomplished by a furnace process.

During the chemical transformation to the inorganic coating of the vascular scaffolds according to the invention, the surface of the magnesium alloy is transformed by a chemical reaction into the desired magnesium fluoride coating in the presence of a fluoride-containing aqueous solution. Depending on the composition of the initial alloy, the coating may contain dysprosium, europium, neodymium, zinc, zirconium, yttrium, erbium, gadolinium and calcium. Furthermore, the coatings may include Si, Ni, Fe, Cu as well as other metals and non-metals due to unavoidable impurities of the magnesium alloy used. Preferred reaction media are hydrofluoric acid, aqueous potassium fluoride solution and aqueous ammonium fluoride solution.

Electrochemical plasma oxidation and the sol-gel process are also suitable methods to produce an inorganic coating according to the invention. In plasma oxidation, oxide layers of up to 10 µm thickness are produced by applying voltages of up to several 100 V in aqueous electrolytes on the surface of the vascular scaffold.

Sol-gel processes involve the formation of a colloidal suspension of so-called precursors. The starting materials for sol synthesis are often alcoholates of metals or non-metals. After the colloidal suspension has been formed, water, an acid, a base or a combination thereof is added to initiate hydrolysis and condensation. The hydrolysis of precursor molecules and the condensation between the resulting reactive species are the essential basic reactions of the sol-gel process. The processes involved and the properties of the precursor molecules have a decisive effect on the resulting material properties. After the coating has been applied to at least a part of the vascular scaffold, the coating composition is heated, which is necessary for aging and removal of organic solvents.

The inorganic coating should be performed relatively uniformly. The inorganic coating can have a thickness of about 0.1 pm to 10 µm and preferably a thickness of 1 pm to about 100 nm, further preferred from 10 pm to about 1 nm. The desired layer thickness also depends on the respective inorganic compound, which may be contained in the inorganic coating in addition to magnesium fluoride, and can be achieved by repeated coating steps interrupted by drying steps. In particular, if the inorganic coating is deposited from a gas phase, the coating becomes impermeable over a longer period of time. In case of short coating times, leaks occur which permit the diffusion of water or gases.

In a suitable solvent, possibly also together with the inorganic compound, the at least one antiinflammatory, antiproliferative, antiangiogenic, antirestenotic (anti-restenosis), antineoplastic, antimigrative and/or antithrombogenic active agent to be applied can be dissolved, emulsified, suspended, or dispersed. If a matrix or carrier substance is contained in the active agent layer, it can be dissolved and applied together with the active agent, or applied separately, preferably previously, in a spraying, pipetting or dipping process.

In a preferred embodiment, the inorganic coating is initially applied to the vascular scaffold, dried, then an organic coating is applied to this coating, dried and finally an active agent is applied. A solution of at least one active agent and possibly a matrix or carrier substance in a volatile solvent is preferably applied to the organic coating of the vascular scaffold. The solvent or solvent mixture is then removed by evaporation at room temperature.

Suitable solvents are water and preferably organic solvents such as chloroform, methylene chloride (dichloromethane), acetone, tetrahydrofuran (THF), diethyl ether, methanol, ethanol, propanol, isopropanol, diethyl ketone, dimethylformamide (DMF), dimethylacetamide, ethyl acetic acid ester, dimethyl sulfoxide (DMSO), benzene, toluene, xylene, t-butylmethylether (MTBE), petroleum ether (PE), cyclohexane, pentane, hexane, heptane, wherein chloroform and methylene chloride are particularly preferred.

Another preferred embodiment of the vascular scaffolds, according to the invention has three coatings. In such three-layer systems, the first coating is the one applied directly to the vascular scaffold. The second coating is the coating that is applied to this first coating. The coating applied to the second coating is referred to as the third coating.

According to the three-layer implementation, the first coating consists of a pure inorganic coating comprising magnesium fluoride coated with a second coating containing at least one organic polymer or consisting of this polymer only. The second coating is covered by a third coating containing at least one antiproliferative, antiphlogistic and/or antithrombogenic active agent, i.e. an antirestenotic active agent or consisting of this active agent only.

Instead of this three-layer assembly, the outer active agent layer can also be omitted, and the active agent can be embedded in the organic coating. Thus, in another preferred embodiment, the inorganic coating is initially applied to the vascular scaffold, dried, then an organic coating together with an active agent is applied to this coating and dried.

Of course, it is also possible to apply the active agent as an outer third layer to the middle organic layer containing the active agent. The middle organic layer may contain the same active agent in a different or the same concentration or a different active agent or no active agent is used in the middle organic layer.

A preferred embodiment of the invention thus comprises of a vascular scaffold consisting of a scaffold body of one of the herein disclosed biodegradable magnesium alloys, an inorganic coating comprising magnesium fluoride, an organic coating and a polymeric coating, optionally at least one antirestenotic agent.

If the at least one active agent or combination of active agents is applied to the polymeric coating of the vascular scaffold, further substances can be applied in combination with the at least one active agent or combination of active agents as pharmacologically acceptable carriers or as a matrix.

The already above mentioned polymers as wells as low molecular substances may serve as pharmacologically acceptable carriers, such as for example lactose, starch, sodium carboxymethyl starch, sorbitol, sucrose, magnesium stearate, dicalcium phosphate, calcium sulfate, talcum, mannitol, ethyl alcohol, polyvinyl alcohols, polyvinyl pyrrolidone, gelatine, naturally occurring sugars, naturally occurring as well as synthetic gums such as acacia gum or guar gum, sodium alginate, sodium benzoate, sodium acetate, glycerides, isopropyl myristates and palmitates, citrates, such as tributyl and triethyl citrates and their acetyl derivatives, phthalates such as dimethyl phthalate or dibutyl phthalate, etc. benzoic acid benzyl ester, triacetine, 2-pyrrolidone, boric acid, magnesium aluminium silicates, naturally occurring carob gum, gum karaya, guar, tragacanth, agar, cellulose, cellulose derivatives such as methyl cellulose, sodium carboxymethyl cellulose, hydroxypropyl methyl cellulose, microcrystalline cellulose as well as alginates, aluminas and bentonites, polyethylene glycol and also waxes such as for example beeswax, carnauba wax, candelilla wax and the like can be used. The matrix substance of the second layer can be identical to the polymer of the first layer. The additional carrier or matrix substances can be used in a weight ratio of up to 70% by wt., preferably to 50% by wt. based on the used active agent(s).

By means of known methods such as spraying, dipping, plasma, brushing, syringe, electrospinning or pipetting methods the polymeric coating is applied to the inorganic coating, which is already contained on the magnesium alloy of the basic scaffold, and preferably also adheres firmly to it. Thus, the vascular scaffold according to the invention may be coated by spraying, pipetting, brushing, injection, plasma deposition or immersion methods, electrospinning, wherein the polymeric substance or mixtures of substances are dissolved in a solvent and this solution is applied to the implant. The solvent or solvent mixture is then removed by evaporation at room temperature. The coating of the vascular scaffolds according to the invention can be performed both before and after crimping onto a catheter balloon. If the coating is at first applied after the vascular scaffold was attached to a catheter balloon, a dipping or spraying process is preferred. Thereby, the catheter balloon may possibly also be coated beyond the ends of the vascular scaffold. The polymer can also be preformed in form of a tube and applied to the outer or inner surface of the basic scaffold of the vascular scaffolds according to the invention provided with the inorganic coating. If a tube is applied or the polymeric coating is applied as a full-surface coating, i.e. a coating covering the entire interspace, it is preferred that this polymeric coating extends beyond the length of the vascular scaffold or the vascular graft and does not terminate with the ends of the vascular graft. In a further step, the protruding ends of the coating are placed to the outside around the edges of the vascular graft and the resulting edges are integrated into the underlying polymer layer under pressure and increased temperature. This ensures a reinforced coating at the vascular scaffold ends and reduces the risk of detachment at these weak points.

The polymeric coating should be preformed relatively uniformly and should have a layer thickness of 0.01 to 100 µm. The desired layer thickness also depends on the respective polymer and can be achieved by multiple coating steps interrupted by drying steps. The coating thickness can be used to adjust the density of the polymeric coating. Particularly when the polymer is deposited from a gas phase, the coating becomes impermeable over a longer coating period. In case of short coating times, leaks occur that permit the diffusion of water or gases. Layer thicknesses of the polymeric coating from 0.01 to 90 µm are particularly preferred, further preferred from 0.01 to 80 µm, further preferred from 0.01 to 70 µm, further preferred from 0.01 to 60 µm, further preferred from 0.01 to 50 µm, further preferred from 0,01 to 40 µm, more preferred from 0.01 to 30 µm, more preferred from 0.01 to 20 µm, more preferred from 0.01 to 10 µm, more preferred from 0.05 to 10 µm, more preferred from 0.1 to 10 µm and most preferred from 0.5 to 10 µm.

Suitable solvents are water and preferably organic solvents such as chloroform, methylene chloride (dichloromethane), acetone, tetrahydro-furan (THF), diethyl ether, methanol, ethanol, propanol, isopropanol, diethyl ketone, Dimethylformamide (DMF), dimethylacetamide, ethyl acetic acid ester, dimethyl sulfoxide (DMSO), benzene, toluene, xylene, t-butylmethylether (MTBE), petroleum ether (PE), cyclohexane, pentane, hexane, heptane, wherein chloroform and methylene chloride are particularly preferred.

In a suitable solvent or also together with the polymer, the at least one antiinflammatory, anti proliferative, antiangiogenic, antirestenotic (anti-restenosis), antineoplastic, antimigrative and/or antithrombogenic active agent to be applied can also be dissolved, emulsified, suspended or dispersed. If a polymer is contained as a matrix substance in the organic coating, this polymer can be dissolved and applied together with the active agent, or applied separately, preferably previously, in a spraying, pipetting or dipping process.

In a preferred embodiment, inorganic coating is initially applied to the scaffold body, then the polymeric coating is applied to the inorganic coating, dried, and then an active agent is applied to the polymeric coating. A solution of at least one active agent and possibly a carrier substance in a volatile solvent is preferably applied to the polymeric coating of the vascular scaffolds. The solvent or solvent mixture is then removed by evaporation at room temperature.

Another aspect of the present invention is directed to a **method for producing a vascular scaffold** as described herein. Thus, the present invention is also directed to a method for producing a vascular scaffold, comprising the steps:
A) providing a vascular scaffold made of a biodegradable magnesium alloy comprising a cylindrical scaffold body formed of interconnected struts, wherein the interconnected struts comprise at least one structure designed to hold at least one radiopaque marker;
B) joining at least one radiopaque marker into the at least one structure of the vascular scaffold thereby establishing a form-fit or force-fit connection between the at least one radiopaque marker and the at least one structure,
C) selectively, at least partially laser melting the at least one radiopaque marker in the at least one structure of the vascular scaffold, and
D) applying an inorganic coating comprising magnesium fluoride to the vascular scaffold,

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

The vascular scaffold in step A) can be obtained by any conventional means for producing such scaffolds. Preferably, the cylindrical scaffold body, including the interconnected struts and the at least one structure is prepared from a solid tube made of a biodegradable magnesium alloy by laser cutting. The scaffold structure and the at least one structure for holding a radiopaque marker can be cut out in the same step from the solid tube, such that no additional step is required for forming the at least one structure at the scaffold body.

In step B) of the method for producing a vascular scaffold as described herein, the radiopaque marker can be joined by any suitable means into the at least one structure, thereby establishing a form-fit or force-fit connection, including pressing, crimping, or riveting. Preferably, the radiopaque marker is crimped into the at least one structure. To this extent, suitable tools (special punches, tweezers, crimping tools) may be used. Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) crimping at least one radiopaque marker into the at least one structure of the vascular scaffold thereby establishing a form-fit or force-fit connection between the radiopaque marker and the at least one structure,
C) selectively, at least partially laser melting the at least one radiopaque marker in the at least one structure of the vascular scaffold, and
D) applying an inorganic coating comprising magnesium fluoride to the vascular scaffold,

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In a preferred embodiment, in step C) the joined radiopaque marker is laser melted at, so that the liquefied material adapts better to the contour of the at least one structure, thereby improving the form-fit or force-fit connection between the radiopaque marker and the at least one structure. Selectively melting means herein, that only the marker is melted and not the scaffold, and can be performed by any suitable means, including laser welding and laser melting. Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold, and
D) applying an inorganic coating comprising magnesium fluoride to the vascular scaffold,

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) crimping at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold, and
D) applying an inorganic coating comprising magnesium fluoride to the vascular scaffold,

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In a further embodiment of the production method, the scaffold with the radiopaque marker inserted is polished to the target wall thickness. Due to form-fit or force-fit connection between the radiopaque marker and the at least one structure, the radiopaque marker is so firmly bonded to the scaffold that it does not detach during polishing procedure. Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D1) polishing the vascular scaffold obtained in step C to a defined target thickness
D2) applying an inorganic coating comprising magnesium fluoride to the vascular scaffold,
wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D1) electropolishing the vascular scaffold obtained in step C to a defined target thickness
D2) applying an inorganic coating comprising magnesium fluoride to the vascular scaffold,
wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) crimping at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D1) electropolishing the vascular scaffold obtained in step C to a defined target thickness
D2) applying an inorganic coating comprising magnesium fluoride to the vascular scaffold,
wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In step D) or D2) the inorganic coating can be applied by any suitable means, including the coating methods described herein. Preferably, the inorganic coating is applied by passivating the vascular scaffold obtained in step C) in hydrofluoric acid. Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold, and
D) passivating the vascular scaffold obtained in step B) in hydrofluoric acid,

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In another embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D1) electropolishing the vascular scaffold obtained in step B2) to a defined target thickness, and
D2) passivating the vascular scaffold obtained in step B) in hydrofluoric acid,
wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In a further embodiment, the method for producing a vascular scaffold comprises step E):
E) applying an organic coating to the vascular scaffold.

The organic coating may be either applied directly onto the vascular scaffold or on an underlying inorganic coating. Any suitable coating method as described herein may be used, including spraying, dipping, plasma, brushing, syringe or pipetting method.

Thus, in a preferred embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D) applying an inorganic coating comprising magnesium fluoride to the vascular scaffold, and
E) applying an organic coating to the vascular scaffold

wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner,
wherein the at least one radiopaque marker is dysprosium.

In another embodiment, the method for producing a vascular scaffold, comprises the steps:
A) providing a vascular scaffold made of a biodegradable magnesium alloy comprising a cylindrical scaffold body formed of interconnected struts, wherein the interconnected struts comprise at least one structure designed to hold a radiopaque marker;
B) joining at least one radiopaque marker into the at least one structure of the vascular scaffold thereby establishing a form-fit or force-fit connection between the at least one radiopaque marker and the at least one structure,
C) selectively laser melting the at least one radiopaque marker in the at least one structure of the vascular scaffold,
D1) electropolishing the vascular scaffold obtained in step C) to a defined target thickness,
D2) optionally, passivating the vascular scaffold obtained in step D1) in hydrofluoric acid,
E) applying an organic coating to the vascular scaffold,
wherein the vascular scaffold is made of a biodegradable magnesium alloy and comprises a cylindrical scaffold body formed of interconnected struts, wherein the interconnected struts comprise at least one structure designed to hold a radiopaque marker and wherein the at least one structure holds a rare-earth metal as radiopaque marker in a form-fitting or force-fitting manner, and wherein the at least one radiopaque marker is dysprosium.

### Description of the figures

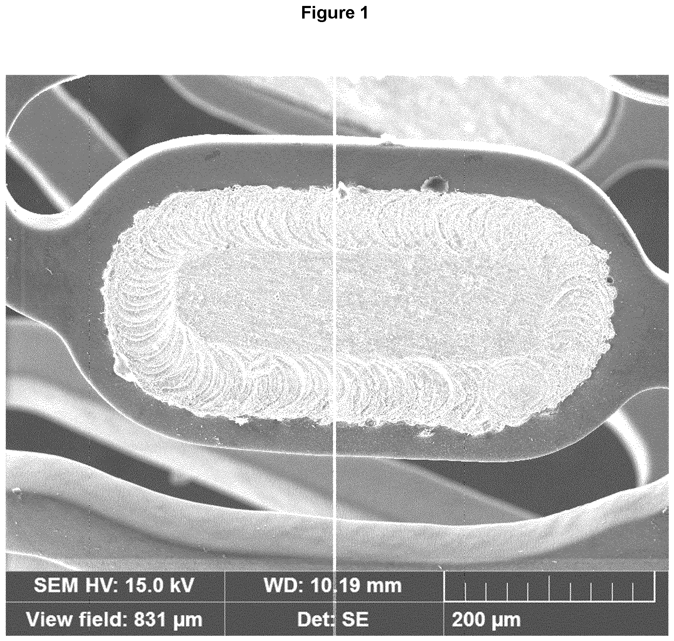

**Figure 1** shows a REM image of the inventive vascular scaffold comprising at least one structure holding dysprosium as radiopaque marker, wherein the dysprosium marker has the shape of an oval disc with a width of 600 µm and a height of 250 µm.
**Figure 2** shows a comparison of the photometric measurements to the degradation rates of the uncoated scaffold without marker (bare metal stent (BMS)), uncoated scaffold with dysprosium marker, uncoated scaffold with gadolinium marker, uncoated scaffold with yttrium marker and uncoated scaffold with neodymium marker.
**Figure 3** shows a comparison of the photometric measurements to the degradation rates of the uncoated scaffold without marker (bare metal stent (BMS)), fluoridated scaffold with dysprosium marker, fluoridated scaffold with gadolinium marker, fluorinated scaffold with yttrium marker and fluoridated scaffold with neodymium marker.
**Figure 4** shows camera images of the degradation of individual uncoated and fluoridated scaffolds with dysprosium marker, gadolinium marker, yttrium marker and neodymium marker at (**A**) t=0, 6 hours, and 12 hours; (**B**) t=18 hours, 24 hours, and 48 hours.
**Figure 5** shows camera images of the degradation of a PLGA coated scaffold with dysprosium marker, at t=0, 12 hours, 24 hours, 36 hours, 48 hours, and 72 hours.
**Figure 6** shows REM images of uncoated scaffolds (left) and fluoridated scaffolds (right) comprising a dysprosium marker, gadolinium marker, yttrium marker or a neodymium marker.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice.

### Examples

### Example 1: Preparation of an inventive vascular scaffold bearing a radiopaque marker

1. Manufacturing a vascular scaffold comprising at least one structure by laser cutting.
2. A semi-finished product of the radiopaque marker (tube or sheet metal) is selected depending on the target wall thickness of the polished scaffold. For example: If the scaffold has a final wall thickness of 100 µm, a sheet or tube of <= 100 µm of the radiopaque marker material is selected.
3. Laser cutting: The radiopaque markers are cut by laser according to the contour of the structure of the previously laser-cut scaffold, required for forming a press fit connection.
4. Pressing/Crimping/Joining: The individual markers are pressed or crimped into the intended eyelet in the scaffold using suitable tools (special punches, tweezers, crimping tools) so that a form-fit connection is created.
5. Laser welding or laser melting: The markers can be melted onto the surface using a laser so that the liquefied material adapts even better to the contour of the eyelet of the scaffold. Only the marker material is liquefied, not the scaffold material.
6. Electropolishing: The scaffold with the marker inserted is polished to the target wall thickness. The marker is so firmly bonded to the scaffold that it does not come loose during electropolishing without abrasion of marker material.
7. Inorganic coating: The scaffold and markers can then be subjected to heat treatment and/or passivation using hydrofluoric acid.
8. Optionally coating: the scaffold and markers (passivated or non-passivated) can be coated with an organic polymer coating and a anti proliferative drug.

### Reference Example 2: Degradation behaviour of a vascular scaffold comprising a radiopaque marker without an inorganic coating.

The coated and uncoated scaffolds of example 1 comprising dysprosium, gadolinium, yttrium or neodymium marker were examined in a degradation test.

The degradation tests were carried out with a degradation test machine (DTM) from MeKo Manufacturing e.K. The DTM is equipped with a peristaltic pump, a temperature sensor, heating system, flow sensor and a camera system.

The vascular scaffolds used for the tests were manufactured by MeKo Manufacturing e.K. The scaffold material is a magnesium alloy (Mg10Dy1Nd1Zn0.2Zr).

The filling quantity of the test fluid PBS (phosphate buffered saline) was 500 ml. The temperature of the fluid is maintained at 37±2°C. The flow rate [ml/min], the temperature of the fluid [°C] and the digital images [1 image/min] are continuously recorded and stored by the system. The test fluid PBS consists of: 8.0 [g/l] NaCl, 0.2 [g/l] KCI, 1.15 [g/l] Na₂HPO₄ and 0.2 [g/l] KH₂PO₄.

The pH values were measured at different times by using the Mettler Toledo SevenGo SG2, Serial No. B426752831 measuring instrument and the Mettler Toledo InLab 413 SG/2m IP67, No. 51340288 measuring probe.

### Photometry Measurement

The photometry measurement for the concentration measurement of the magnesium concentrations over the test period was carried out with a spectrophotometer.

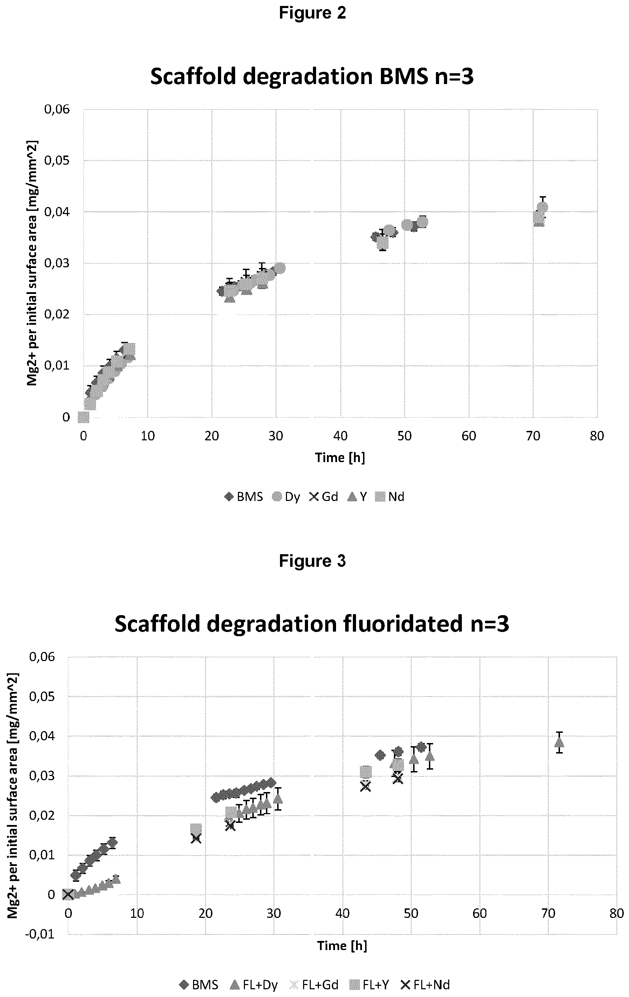

The measurement results are shown graphically in Figure 2 and Figure 3. The photometric concentration measurements showed no significant differences between scaffolds with or without markers dysprosium, gadolinium, yttrium or neodymium. A difference is only recognizable for the respective fluoridated scaffolds of each group: The fluoridated samples degrade significantly slower than the uncoated samples, especially in the first 20 hours (~3.5 vs. 13 µg/mm² after 6.5 hours).

According to the concentration measurements, the uncoated BMS degraded at the same rate as the uncoated BMS without a marker.

### Camera shots

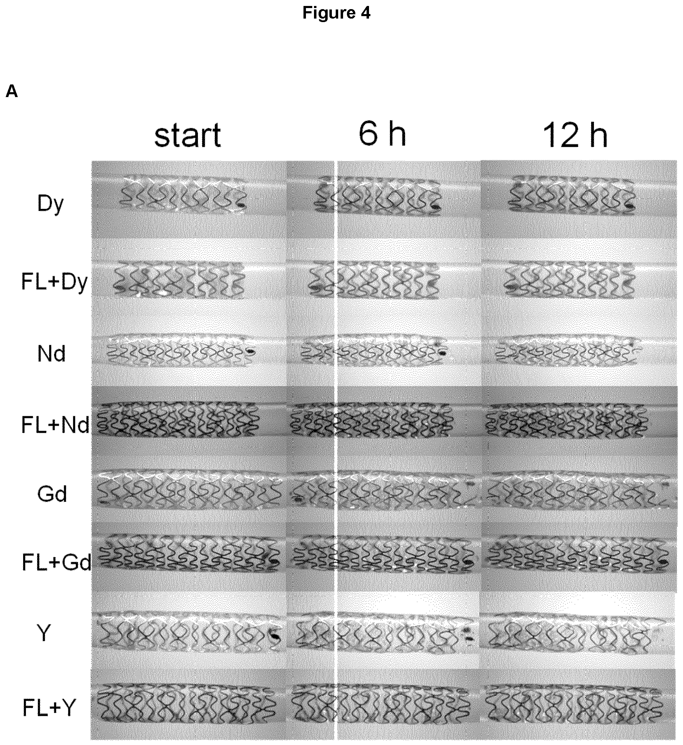

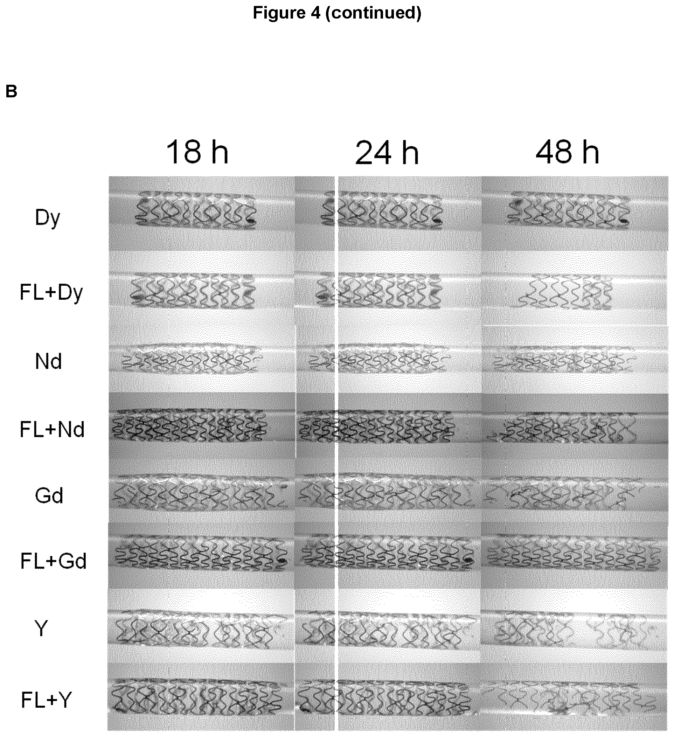

The images in Figure 4 confirm the photometric measurements. Figure 4 shows one scaffold of each group. It is noticeable that the markers or marker eyelets in groups Y, Gd and Nd degrade significantly faster than the rest of the respective scaffold. This has not been observed for Dy containing scaffolds. Here, the marker eyelet does not degrade noticeably faster than the rest of the scaffold.

A more rapid degradation of the marker eyelets was not observed for the fluoridated scaffolds.

It is noteworthy that compared to the overall degradation of the scaffold, the degradation of the marker eyelets cannot be traced by a change in concentration, as the marker degradation only make up a very small proportion of the total volume. The concentration measurement method is not sensitive enough for such scaffolds.

### Conclusion

Fluoridation slows down the overall degradation as well as the degradation of the marker eyelets regardless of the marker material, whereby it must be mentioned that the Gd markers were no longer firmly seated in the eyelets and could presumably be rinsed out (see Figure 4A and Figure 4B). The Nd markers were no longer present after fluoridation.

All BMS degraded significantly faster in the area of the markers than the rest of the scaffold (see Fig. Figure 4A and Figure 4B), except scaffolds with dysprosium marker.

Scaffolds with dysprosium marker did not show an accelerated degradation of the marker eyelet, thereby rendering dysprosium as an extraordinarily compatible marker material with regard to the degradation of the surrounding scaffold.

### Reference Example 3: Degradation behaviour of a vascular scaffold comprising a radiopaque marker with a polymer coating and without an inorganic coating.

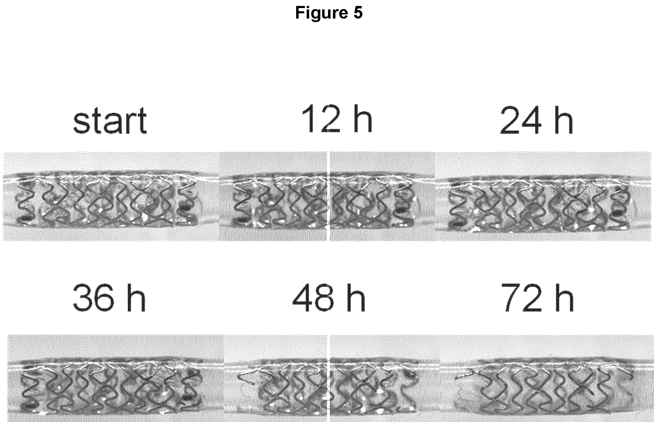

Scaffolds having a Dy marker and without an inorganic coating were prepared in the same way as in Example 1, but with a subsequent coating of PLGA using ultrasonic spray coating. The coating was applied over the entire surface of the scaffold and the marker, i.e. without any imperfections. The degradation test was carried out in accordance with Example 2. Images of the degraded scaffolds are shown in Figure 5.

### Results

Figure 5 shows a premature degradation of the dysprosium marker eyelets despite a full-surface coating of PLGA.

An organic coating alone does not provide sufficient protection to stop or slow down galvanic corrosion.

### Reference Example 4: Attempts to passivate the vascular scaffolds having different radiopaque markers with hydrofluoric acid.

Scaffolds with different radiopaque markers were prepared according to example 1 and passivated with hydrofluoric acid.

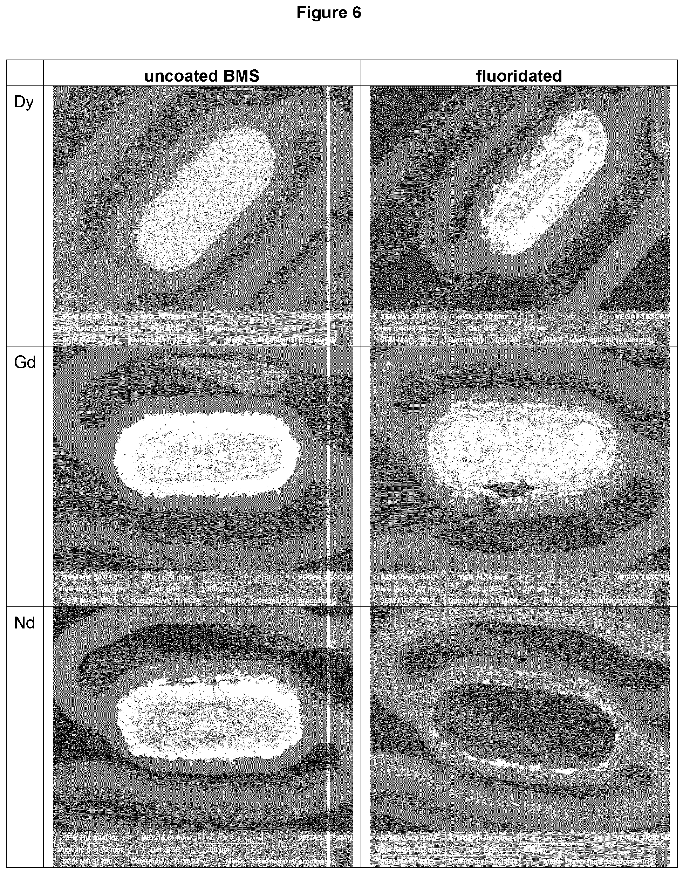

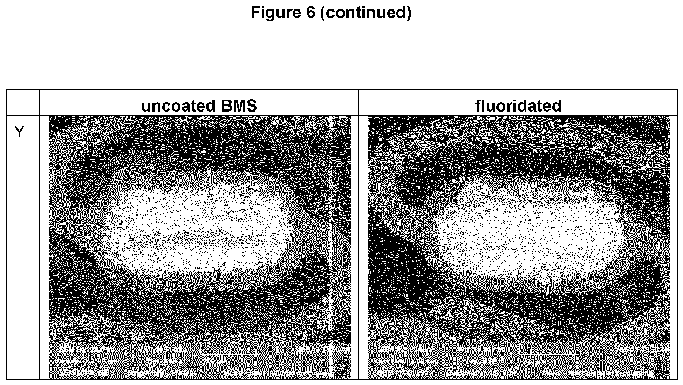

Figure 6 shows images of the marker eyelets after passivation.

### Results

### Dysprosium marker

Scaffolds with dysprosium marker could be fluoridated without residue release of particle formation. The marker showed no observable volume increase after fluoridation. The marker sits tight in the eyelet before and after fluoridation. No loosening or release of the marker was observed.

### Gadolinium marker.

The gadolinium marker showed a significant volume increase after fluoridation, which caused stress fractures and cracks in the marker eyelet. The marker also exhibited an extremely rough surface after fluoridation. Further, marker particles were observed that have been detached from the scaffold, thereby rendering gadolinium not suitable as a marker material for fluoridated scaffolds.

It is noteworthy, that prior fluoridation, the gadolinium marker sits firmly in the eyelet and is also weldable.

### Neodymium marker:

The neodymium marker dissolved in hydrofluoric acid during passivation which renders neodymium unsuitable as marker material for fluoridated scaffolds. Further, marker particles were observed that have been detached from the scaffold, which is problematic during welding.

### Yttrium marker

Scaffolds with yttrium marker could be fluoridated without residue release of particle formation. The marker showed no observable volume increase after fluoridation. The marker sits tight in the eyelet before and after fluoridation. No loosening or release of the marker was observed. However, Yttrium only has a low mass attenuation coefficient compared to dysprosium and is thus not suitable as a marker material.

## Claims

1. A vascular scaffold made of a biodegradable magnesium alloy comprising a cylindrical scaffold body formed of interconnected struts, wherein the interconnected struts comprise at least one structure designed to hold at least one radiopaque marker, wherein the at least one structure holds at least one radiopaque marker in a form-fitting or force-fitting manner, wherein the at least one radiopaque marker is dysprosium, and wherein the vascular scaffold is completely covered with an inorganic coating comprising magnesium fluoride.

2. The vascular scaffold according to claim 1, wherein the at least one radiopaque marker is pure dysprosium.

3. The vascular scaffold according to claim 1 or 2, wherein the at least one structure is designed as a hole, a blind hole, a through hole, a depression or an eyelet.

4. The vascular scaffold according to any one of claims 1 to 3, wherein the at least one structure is adapted to hold a radiopaque marker having a diameter between 20 µm and 1000 µm and the at least one structure is adapted to hold a radiopaque marker having a thickness between 50 µm and 500 µm.

5. The vascular scaffold according to any one of claims 1 to 4, wherein the inorganic coating comprises magnesium fluoride and a rare-earth metal fluoride, thereby preventing galvanic corrosion of the cylindrical scaffold.

6. The vascular scaffold according to any one of claims 1 to 5, wherein the biodegradable magnesium alloy comprises dysprosium.

7. The vascular scaffold according to any one of claims 1 to 6, wherein the biodegradable magnesium alloy comprises
| | |
|---|---|
| 0.1% by wt. - 15.5% by wt. | dysprosium |
| 0.01% by wt. - 1.5% by wt. | neodymium and/or europium |
| 0.0% by wt. - 2.0% by wt. | zinc |
| 0.0% by wt. - 1.0% by wt. | zirconium |
| at least 80.0% by wt. | magnesium. |

8. The vascular scaffold according to any one of claims 1 to 7, wherein a first structure of the at least one structure holding a radiopaque marker is located at the distal end of the scaffold and a second structure of the at least one structure holding a radiopaque marker is located at the proximal end of the scaffold.

9. The vascular scaffold according to any one of claims 1 to 8, further comprising an organic coating, wherein the organic coating comprises or consists of one or more substances of the following group:
polydopamine, polyvinyl pyrrolidone, glycerol, poly hydroxyethyl methacrylates, polyethylene glycol, polypropylene glycol, polyvinyl alcohol polydioxanone, polycaprolactone, polygluconate, poly(lactic acid) polyethylene oxide copolymer, modified cellulose, polyhydroxybutyrate, polyamino acids, polyphosphate esters, polyvalerolactones, poly-ε-decalactones, polylactonic acid, poly(glycolic acid), polylactides, preferably poly(L-lactide), poly(D,L-lactide), and copolymers as well as blends such as poly(L-lactide-co-glycolide), poly(D,L-lactide-co-glycolide), poly(L-lactide-co-D,L-lactide), poly(L-lactide-co-trimethylene carbonate)] (PTMC), polyglycolides, copolymers of polylactides and polyglycolides, poly(ε-caprolactone), polyhydroxybutyric acid, polyhydroxybutyrates, polyhydroxyvalerates, polyhydroxybutyrate-co-valerates, poly(1,4-dioxane-2,3-dione), poly(1,3-dioxane-2-one), poly(para-dioxanones), polyanhydrides, poly(maleic anhydrides), polyhydroxy methacrylates, fibrin, polycyanoacrylates, polycaprolactone dimethylacrylates, poly-b-maleic acid polycaprolactone butyl acrylates, multiblock polymers of oligocaprolactone diols and oligodioxanone diols, poly(ether ester) multiblock polymers of PEG and poly(butylene terephthalate), polypivalolactone, polyglycolic acid-trimethylene carbonates, polycaprolactone glycolides, poly(g-ethyl glutamate), poly(DTH iminocarbonate), poly(DTE-co-DT-carbonate), poly(bisphenol A iminocarbonate), poly(ortho ester), polyglycolic acid-trimethylene carbonate, poly(trimethylene carbonate) poly(imino carbonate), poly(N-vinylpyrrolidone), polyvinyl alcohols, polyesteramides, glycolized polyesters, polyphosphoesters, polyphosphazenes, poly[(p-carboxyphenoxy) propane], poly(hydroxypentanoic acid), polyanhydrides, poly(ethylene oxide)-poly(propylene oxide), soft polyurethanes, polyurethanes with amino acid residues in the backbone, polyether esters such as polyethylene oxide, polyalkene oxalates, polyorthoesters and their copolymers, lipids, waxes, oils, polyunsaturated fatty acids, eicosapentaenoic acid, timnodonic acid, docosahexaenoic acid, arachidonic acid, linoleic acid, α-linolenic acid, γ-linolenic acid, carrageenans, fibrinogen, agar-agar, starch, collagen, protein-based polymers, polyamino acids, synthetic polyamino acids, Zein, polyhydroxyalkanoates, pectic acid, actinic acid, carboxymethyl sulfate, albumin, hyaluronic acid, chitosan and derivatives thereof, heparan sulfate and derivatives thereof, heparins, chondroitin sulfate, dextran, β-cyclodextrins, copolymers with PEG and polypropylene glycol, gum arabic, guar, gelatin, collagen, collagen-N-hydroxysuccinimide, lipids, phospholipids, polyacrylic acid, polyacrylates, poly(methyl methacrylate), poly(butyl methacrylate), polyacrylamide, polyacrylonitrile, polyamides, polyetheramides, polyethylene amine, polyimides, polycarbonates, polycarbourethanes, polyvinyl ketones, polyvinyl halides, polyvinylidene halides, polyvinyl ethers, polyisobutylenes, polyvinyl aromatic compounds, polyvinyl esters, polyvinylpyrrolidones, polyoxymethylenes, polytetramethylene oxide, polyethylene, polypropylene, polytetrafluoroethylene, polyurethanes, polyetherurethanes, silicone polyetherurethanes, silicone polyurethanes, silicone polycarbonate urethanes, polyolefin elastomers, polyisobutylenes, fluorosilicones, carboxymethylchitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halides, cellulose ethers, cellulose triacetates, shellac, parylene (poly-para-xylylene), parylene N, parylene C and/or parylene D, and copolymers of the aforementioned polymers.

10. The vascular scaffold according to claim 9, wherein at least one antiinflammatory, antiproliferative, antiangiogenic, antirestenotic, antineoplastic, antimigrative and/or antithrombogenic active agent is present in or on the organic coating.

11. A method for producing a vascular scaffold according to any one of claims 1 to 10, comprising the steps:
A) providing a vascular scaffold made of a biodegradable magnesium alloy comprising a cylindrical scaffold body formed of interconnected struts, wherein the interconnected struts comprise at least one structure designed to hold at least one radiopaque marker;
B) joining at least one radiopaque marker into the at least one structure of the vascular scaffold thereby establishing a form-fit or force-fit connection between the at least one radiopaque marker and the at least one structure;
C) selectively, at least partially laser melting the at least one radiopaque marker in the at least one structure of the vascular scaffold, and
D) applying an inorganic coating comprising magnesium fluoride to the vascular scaffold,
wherein the at least one radiopaque marker is dysprosium.

12. The method according to claim 11, wherein the at least one radiopaque marker is pure dysprosium.

13. The method according to claim 11 or 12, further comprising step E):
E) applying an organic coating to the vascular scaffold.

14. The method according to claim 13, wherein at least one antiinflammatory, antiproliferative, antiangiogenic, antirestenotic, antineoplastic, antimigrative and/or antithrombogenic active agent is present in or on the organic coating.

## Patentansprüche

1. Ein vaskuläres Gerüst aus einer biologisch abbaubaren Magnesiumlegierung bestehend aus einem zylindrischen Gerüstkörper, der aus miteinander verbundenen Streben gebildet ist, wobei die miteinander verbundenen Streben mindestens eine Struktur aufweisen, die dazu ausgelegt ist, mindestens einen röntgendichten Marker zu halten, wobei die mindestens eine Struktur mindestens einen röntgendichten Marker formschlüssig oder kraftschlüssig hält, wobei der mindestens eine röntgendichte Marker Dysprosium ist, und wobei das vaskuläre Gerüst vollständig mit einer anorganischen Beschichtung aus Magnesiumfluorid bedeckt ist.

2. Das vaskuläre Gerüst gemäß Anspruch 1, wobei der mindestens eine röntgendichte Marker reines Dysprosium ist.

3. Das vaskuläre Gerüst gemäß Anspruch 1 oder 2, wobei die mindestens eine Struktur als Loch, Sackloch, Durchgangsloch, Vertiefung oder Öse ausgebildet ist.

4. Das vaskuläre Gerüst gemäß einem der Ansprüche 1 bis 3, wobei die mindestens eine Struktur dazu ausgelegt ist, einen röntgendichten Marker mit einem Durchmesser zwischen 20 µm und 1000 µm zu halten, und die mindestens eine Struktur dazu ausgelegt ist, einen röntgendichten Marker mit einer Dicke zwischen 50 µm und 500 µm zu halten.

5. Das vaskuläre Gerüst gemäß einem der Ansprüche 1 bis 4, wobei die anorganische Beschichtung Magnesiumfluorid und ein Seltenerdmetallfluorid umfasst, wodurch eine galvanische Korrosion des zylindrischen Gerüsts verhindert wird.

6. Das vaskuläre Gerüst gemäß einem der Ansprüche 1 bis 5, wobei die biologisch abbaubare Magnesiumlegierung Dysprosium umfasst.

7. Das vaskuläre Gerüst gemäß einem der Ansprüche 1 bis 6, die biologisch abbaubare Magnesiumlegierung umfassend
| | |
|---|---|
| 0,1 Gew.-% - 15,5 Gew.-% | Dysprosium |
| 0,01 Gew.-% - 1,5 Gew.-% | Neodym und/oder Europium |
| 0,0 Gew.-% - 2,0 Gew.-% | Zink |
| 0,0 Gew.-% - 1,0 Gew.-% | Zirkonium |
| mindestens 80,0 Gew.-% | Magnesium. |

8. Das vaskuläre Gerüst gemäß einem der Ansprüche 1 bis 7, wobei sich eine erste Struktur der mindestens einen Struktur, die einen röntgendichten Marker hält, am distalen Ende des Gerüsts befindet und sich eine zweite Struktur der mindestens einen Struktur, die einen röntgendichten Marker hält, am proximalen Ende des Gerüsts befindet.

9. Das vaskuläre Gerüst gemäß einem der Ansprüche 1 bis 8, weiter umfassend eine organische Beschichtung, wobei die organische Beschichtung eine oder mehrere Substanzen aus der folgenden Gruppe umfasst oder daraus besteht:
Polydopamin, Polyvinylpyrrolidon, Glycerin, Polyhydroxyethylmethacrylate, Polyethylenglykol, Polypropylenglykol, Polyvinylalkohol, Polydioxanon, Polycaprolacton, Polygluconat, Poly(milchsäure), Polyethylenoxid-Copolymer, modifizierte Cellulose, Polyhydroxybutyrat, Polyaminosäuren, Polyphosphatester, Polyvalerolactone, Poly-e-decalactone, Polylactonsäure, Poly(glykolsäure), Polylactide, bevorzugt Poly(L-lactid), Poly(D,L-lactid) und Copolymere sowie Mischungen wie Poly(L-lactid-co-glycolid), Poly(D,L-lactid-co-glycolid), Poly(L-lactid-co-D,L-lactid), Poly(L-lactid-co-trimethylencarbonat)] (PTMC), Polyglycolide, Copolymere aus Polylactiden und Polyglycoliden, Poly(ε-caprolacton), Polyhydroxybuttersäure, Polyhydroxybutyrate, Polyhydroxyvalerate, Polyhydroxybutyrat-Co-Valerate, Poly(1,4-dioxan-2,3-dion), Poly(1,3-dioxan-2-on), Poly(para-dioxanone), Polyanhydride, Poly(maleinsäureanhydride), Polyhydroxymethacrylate, Fibrin, Polycyanoacrylate, Polycaprolactondimethylacrylate, Poly-b-maleinsäurepolycaprolactonbutylacrylate, Multiblockpolymere aus Oligocaprolactondiolen und Oligodioxanondiolen, Poly(etherester)-Multiblockpolymere aus PEG und Poly(butylenterephthalat), Polypivalolacton, Polyglykolsäure-Trimethylencarbonate, Polycaprolactonglycolide, Poly(g-ethylglutamat), Poly(DTH-iminocarbonat), Poly(DTE-co-DT-carbonat), Poly(bisphenol A-iminocarbonat), Poly(orthoester), Polyglykolsäure-Trimethylencarbonat, Poly(trimethylencarbonat), Poly(imino-carbonat), Poly(N-vinylpyrrolidon), Polyvinylalkohole, Polyesteramide, glykolysierte Polyester, Polyphosphoester, Polyphosphazene, Poly[(p-carboxyphenoxy)propan], Poly(hydroxypentansäure), Polyanhydride, Poly(ethylenoxid)-Poly(propylenoxid), weiche Polyurethane, Polyurethane mit Aminosäureresten im Grundgerüst, Polyetherester wie Polyethylenoxid, Polyalkoxalate, Polyorthoester und deren Copolymere, Lipide, Wachse, Öle, mehrfach ungesättigte Fettsäuren, Eicosapentaensäure, Timnodonsäure, Docosahexaensäure, Arachidonsäure, Linolsäure, α-Linolensäure, γ-Linolensäure, Carrageenane, Fibrinogen, Agar-Agar, Stärke, Kollagen, Polymere auf Proteinbasis, Polyaminosäuren, synthetische Polyaminosäuren, Zein, Polyhydroxyalkanoate, Pektinsäure, Actinsäure, Carboxymethylsulfat, Albumin, Hyaluronsäure, Chitosan und Derivate davon, Heparansulfat und Derivate davon, Heparine, Chondroitinsulfat, Dextran, β-Cyclodextrine, Copolymere mit PEG und Polypropylenglykol, Gummi arabicum, Guar, Gelatine, Kollagen, Kollagen-N-hydroxysuccinimid, Lipide, Phospholipide, Polyacrylsäure, Polyacrylate, Poly(methylmethacrylat), Poly(butylmethacrylat), Polyacrylamid, Polyacrylnitril, Polyamide, Polyetheramide, Polyethylenamin, Polyimide, Polycarbonate, Polycarbourethane, Polyvinylketone, Polyvinylhalogenide, Polyvinylidenhalogenide, Polyvinyläther, Polyisobutylene, polyvinylhaltige aromatische Verbindungen, Polyvinylester, Polyvinylpyrrolidone, Polyoxymethylene, Polytetramethylenoxid, Polyethylen, Polypropylen, Polytetrafluorethylen, Polyurethane, Polyetherurethane, Silikonpolyetherurethane, Silikonpolyurethane, Silikonpolycarbonaturethane, Polyolefinelastomere, Polyisobutylene, Fluorsilikone, Carboxymethylchitosane, Polyaryletheretherketone, Polyetheretherketone, Polyethylenterephthalat, Polyvalerate, Carboxymethylcellulose, Cellulose, Rayon, Rayontriacetate, Cellulosenitrate, Celluloseacetate, Hydroxyethylcellulose, Cellulosebutyrat, Celluloseacetatbutyrat, Ethylvinylacetat-Copolymere, Polysulfone, Epoxidharze, ABS-Harze, EPDM-Kautschuke, Silikone wie Polysiloxane, Polydimethylsiloxane, Polyvinylhalogenide, Celluloseether, Cellulosetriacetate, Schellack, Parylen (Poly-para-xylylen), Parylen N, Parylen C und/oder Parylen D und Copolymere der vorgenannten Polymere.

10. Das vaskuläre Gerüst gemäß Anspruch 9, wobei mindestens ein antiinflammatorischer, antiproliferativer, antiangiogener, antirestenotischer, antineoplastischer, antimigrativer und/oder antithrombogener Wirkstoff in oder auf der organischen Beschichtung vorhanden ist.

11. Ein Verfahren zur Herstellung eines vaskulären Gerüsts gemäß einem der Ansprüche 1 bis 10, umfassend die Schritte:
A) Bereitstellen eines vaskulären Gerüsts aus einer biologisch abbaubaren Magnesiumlegierung, das einen zylindrischen Gerüstkörper umfasst, der aus miteinander verbundenen Streben gebildet ist, wobei die miteinander verbundenen Streben mindestens eine Struktur umfassen, die dazu ausgelegt ist, mindestens einen röntgendichten Marker zu halten;
B) Fügen mindestens eines röntgendichten Markers mit der mindestens einen Struktur des vaskulären Gerüsts, wodurch eine formschlüssige oder kraftschlüssige Verbindung zwischen dem mindestens einen röntgendichten Marker und der mindestens einen Struktur hergestellt wird;
C) selektives, zumindest teilweises Laserschmelzen des mindestens einen röntgendichten Markers in der mindestens einen Struktur des vaskulären Gerüsts, und
D) Aufbringen einer anorganischen Beschichtung umfassend Magnesiumfluorid auf das vaskuläre Gerüst,
wobei der mindestens eine röntgendichte Marker Dysprosium ist.

12. Das Verfahren gemäß Anspruch 11, wobei der mindestens eine röntgendichte Marker reines Dysprosium ist.

13. Das Verfahren gemäß Anspruch 11 oder 12, weiter umfassend Schritt E):
E) Aufbringen einer organischen Beschichtung auf das vaskuläre Gerüst.

14. Das Verfahren gemäß Anspruch 13, wobei mindestens ein antiinflammatorischer, antiproliferativer, antiangiogener, antirestenotischer, antineoplastischer, antimigrativer und/oder antithrombogener Wirkstoff in oder auf der organischen Beschichtung vorhanden ist.

## Revendications

1. Une endoprothèse vasculaire en alliage de magnésium biodégradable comprenant un corps cylindrique formé de maillons interconnectés, où les maillons interconnectés comprennent au moins une structure conçue pour contenir au moins un marqueur radio-opaque, où au moins une structure retient au moins un marqueur radio-opaque par complémentarité de forme ou par ajustement serré, où au moins un marqueur radio-opaque est en dysprosium et où l'endoprothèse vasculaire est entièrement recouverte d'un revêtement inorganique comprenant du fluorure de magnésium.

2. L'endoprothèse vasculaire conforme à la revendication 1, où au moins un marqueur radio-opaque est en dysprosium pur.

3. L'endoprothèse vasculaire conforme à la revendication 1 ou 2, où au moins une structure se présente sous la forme d'un trou, d'un trou borgne, d'un trou traversant, d'un évidement ou d'un œillet.

4. L'endoprothèse vasculaire conforme à l'une des revendications 1 à 3, où au moins une structure est adaptée pour contenir un marqueur radio-opaque ayant un diamètre compris entre 20 µm et 1 000 µm et une épaisseur comprise entre 50 µm et 500 µm.

5. L'endoprothèse vasculaire conforme à l'une des revendications 1 à 4, où le revêtement inorganique contient du fluorure de magnésium et un fluorure de métal du groupe des terres rares, empêchant ainsi la corrosion galvanique de l'endoprothèse cylindrique.

6. L'endoprothèse vasculaire conforme à l'une des revendications 1 à 5, où l'alliage de magnésium biodégradable contient du dysprosium.

7. L'endoprothèse vasculaire conforme à l'une des revendications 1 à 6, où l'alliage de magnésium biodégradable contient :
0,1 % en poids à 15,5 % en poids de dysprosium ;
0,01 % en poids à 1,5 % en poids de néodyme et/ou d'europium ;
0,0 % en poids à 2,0 % en poids de zinc ;
0,0 % en poids à 1,0 % en poids de zirconium ;
au moins 80,0 % en poids de magnésium.

8. L'endoprothèse vasculaire conforme à l'une des revendications 1 à 7, où une première structure contenant un marqueur radio-opaque est située à l'extrémité distale de l'endoprothèse et une deuxième structure contenant un marqueur radio-opaque est située à l'extrémité proximale de l'endoprothèse.

9. L'endoprothèse vasculaire conforme à l'une des revendications 1 à 8, comprenant en outre un revêtement organique, lequel contient ou est constitué d'une ou plusieurs substances du groupe suivant :
polydopamine, polyvinylpyrrolidone, glycérol, méthacrylates de polyhydroxyéthyle, polyéthylène glycol, polypropylène glycol, alcool polyvinylique, polydioxanone, polycaprolactone, polygluconate, copolymère polylactique polyoxyde d'éthylène, cellulose modifiée, polyhydroxybutyrate, polyaminoacides, esters de polyphosphate, polyvalérolactones, poly-ε-décalactones, acide polylactonique, acide polyglycolique, polylactides, de préférence poly(L-lactide), poly(D,L-lactide), et copolymères ainsi que mélanges tels que poly(L-lactide -co-glycolide), poly(D,L-lactide-co-glycolide), poly(L-lactide-co-D,L-lactide), poly(L-lactide -co-triméthylène carbonate) (PTMC), polyglycolides, copolymères de polylactides et de polyglycolides, poly(ε-caprolactone), acide polyhydroxybutyrique, polyhydroxybutyrates, polyhydroxyvalérates, polyhydroxybutyrate-co-valérates, poly(1,4-dioxane-2,3-dione), poly(1,3-dioxane-2-one), poly(paradioxanones), polyanhydrides, poly(anhydrides maléiques), polyhydroxy-méthacrylates, fibrine, polycyanoacrylates, polycaprolactone diméthylacrylates, polycaprolactone butyl acrylates d'acide maléique, polymères multiblocs d'oligodiols de caprolactone et d'oligodiols de dioxanone, polymères multiblocs de PEG et de poly(téréphtalate de butylène), poly(éther-ester), polypivalolactone, carbonates de triméthylène d'acide polyglycolique, glycolides de polycaprolactone, poly(g-éthyl glutamate), poly(DTH iminocarbonate), poly(DTE-co-DT-carbonate), poly(bisphénol A iminocarbonate), poly(ortho ester), carbonate de triméthylène d'acide polyglycolique, poly(triméthylène carbonate) poly(iminocarbonate), poly(N-vinylpyrrolidone), alcools polyvinyliques, polyesteramides, polyesters glycolés, polyphosphoesters, polyphosphazènes, poly[(p-carboxyphénoxy) propane], poly(acide hydroxypentanoïque), polyanhydrides, poly(oxyde d'éthylène)-poly(oxyde de propylène), polyuréthanes mous, polyuréthanes avec des résidus d'acides aminés dans la chaîne principale, esters de polyéther tels que oxyde de polyéthylène, oxalates de polyalkène, polyorthoesters et leurs copolymères, lipides, cires, huiles, acides gras polyinsaturés, acide eicosapentaénoïque, acide timnodonique, acide docosahexaénoïque, acide arachidonique, acide linoléique, acide α-linolénique, acide γ-linolénique, carraghénanes, fibrinogène, agar-agar, amidon, collagène, polymères à base de protéines, polyacides aminés, polyacides aminés synthétiques, zéine, polyhydroxyalcanoates, acide pectique, acide actinique, sulfate de carboxyméthyle, albumine, acide hyaluronique, chitosane et ses dérivés, sulfate d'héparane et ses dérivés, héparines, sulfate de chondroïtine, dextran, β-cyclodextrines, copolymères avec PEG et polypropylène glycol, gomme arabique, gomme de guar, gélatine, collagène, collagène-N-hydroxysuccinimide, lipides, phospholipides, acide polyacrylique, polyacrylates, poly(méthacrylate de méthyle), poly(méthacrylate de butyle), polyacrylamide, polyacrylonitrile, polyamides, polyétheramides, polyéthylène amine, polyimides, polycarbonates, polycarbouréthanes, polyvinylcétones, halogénures de polyvinyle, halogénures de polyvinylidène, éthers de polyvinyle, polyisobutylènes, composés aromatiques polyvinyliques, esters polyvinyliques, polyvinylpyrrolidones, polyoxyméthylènes, oxyde de polytétraméthylène, polyéthylène, polypropylène, polytétrafluoroéthylène, polyuréthanes, polyétheruréthanes, polyétheruréthanes de silicone, polyuréthanes de silicone, uréthanes de polycarbonate de silicone, élastomères de polyoléfine, polyisobutylènes, fluorosilicones, carboxyméthylchitosanes, polyarylétheréthercétones, polyétheréthercétones, polyéthylène téréphtalate, polyvalérates, carboxyméthylcellulose, cellulose, rayonne, triacétates de rayonne, nitrates de cellulose, acétates de cellulose, hydroxyéthylcellulose, butyrates de cellulose, butyrates d'acétate de cellulose, copolymères d'éthylène-acétate de vinyle, polysulfones, résines époxy, résines ABS, caoutchoucs EPDM, silicones tels que polysiloxanes, polydiméthylsiloxanes, polyhalogénures de vinyle, éthers de cellulose, triacétates de cellulose, gomme-laque, parylène (poly-para-xylène), parylène N, parylène C et/ou parylène D, ainsi que copolymères des polymères susmentionnés.

10. L'endoprothèse vasculaire conforme à la revendication 9, où au moins un agent actif anti-inflammatoire, antiprolifératif, anti-angiogénique, antiresténotique, antinéoplasique, antimigratif et/ou antithrombogénique est présent dans ou sur le revêtement organique.

11. Une méthode de fabrication d'une endoprothèse vasculaire conforme à l'une des revendications 1 à 10, comprenant les étapes suivantes :
A) réalisation d'une endoprothèse vasculaire en alliage de magnésium biodégradable comprenant un corps cylindrique formé de maillons interconnectés, où les maillons interconnectés comprennent au moins une structure conçue pour contenir au moins un marqueur radio-opaque ;
B) intégration d'au moins un marqueur radio-opaque à au moins une structure de l'endoprothèse vasculaire, établissant ainsi une liaison par complémentarité de forme ou par ajustement serré entre au moins un marqueur radio-opaque et au moins une structure ;
C) fusion sélective au laser, au moins partiellement, d'au moins un marqueur radio-opaque dans au moins une structure de l'endoprothèse vasculaire ;
D) application d'un revêtement inorganique contenant du fluorure de magnésium sur l'endoprothèse vasculaire ;
où au moins un marqueur radio-opaque est du dysprosium.

12. La méthode conforme à la revendication 11, où au moins un marqueur radio-opaque est en dysprosium pur.

13. La méthode conforme à l'une des revendications 11 à 12, comprenant en outre l'étape E) suivante :
E) application d'un revêtement organique sur l'endoprothèse vasculaire.

14. La méthode conforme à la revendication 13, où au moins un agent actif anti-inflammatoire, antiprolifératif, anti-angiogénique, antiresténotique, antinéoplasique, antimigratif et/ou antithrombogénique est présent dans ou sur le revêtement organique.
